(19) 

Europäisches
Patentamt
European
Patent Office
Office européen
des brevets

(11) **EP 4 778 515 A1**

(12) **EUROPEAN PATENT APPLICATION**
published in accordance with Art. 153(4) EPC

(43) Date of publication:
**22.07.2026 Bulletin 2026/30**

(21) Application number: **24864705.9**

(22) Date of filing: **12.09.2024**

(51) International Patent Classification (IPC):
**A61K 9/70** (2006.01)        **A61K 31/519** (2006.01)
**A61P 25/18** (2006.01)

(52) Cooperative Patent Classification (CPC):
**A61K 9/70; A61K 31/519; A61K 47/32;**
**A61K 47/38; A61P 25/00; A61P 25/18**

(86) International application number:
**PCT/CN2024/118533**

(87) International publication number:
**WO 2025/055991 (20.03.2025 Gazette 2025/12)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **14.09.2023  CN 202311186977**

(71) Applicants:
• **Shanghai Aurora Biotechnology Co., Ltd.**
**Shanghai 201203 (CN)**
• **Shanghai Bocimed Pharmaceutical Research**
**Co., Ltd.**
**Shanghai 201203 (CN)**
• **Taizhou Bocimed Pharmaceutical Co., Ltd.**
**Taizhou, Jiangsu 225316 (CN)**

(72) Inventors:
• **YING, Shuhuan**
**Shanghai 201210 (CN)**
• **FU, Jun**
**Shanghai 201210 (CN)**
• **JIN, Haigang**
**Shanghai 201210 (CN)**
• **GUO, Zhen**
**Shanghai 201210 (CN)**
• **WANG, Tingting**
**Shanghai 201210 (CN)**

(74) Representative: **Gille Hrabal**
**Partnerschaftsgesellschaft mbB**
**Patentanwälte**
**Brucknerstraße 20**
**40593 Düsseldorf (DE)**

(54) **RISPERIDONE SOLID ORAL FILM, PREPARATION METHOD THEREFOR AND USE THEREOF**

(57)     A risperidone solid oral film. Each dosage unit of the oral film comprises: 0.10%-8.00% of risperidone, 5.00%-40.00% of a flavoring agent, 5.00%-40.00% of a stabilizer, 2.00%-15.00% of a plasticizer, 0%-45.00% of a filler, 2.00%-8.00% of a coloring agent and 25.00%-70.00% of a film-forming material. The risperidone solid oral film does not contain polyvinylpyrrolidone and a coating material. The risperidone solid oral film is instant and/or quick-release, and thus achieves fast dissolution and a high dissolution rate, and the preparation method involves simple operation and good reproducibility and thus is suitable for industrial production.

RRT1.14 impurity under different citric acid dosages

The dosage of citric acid : 2 mg/tablet
The dosage of citric acid: 4 mg/tablet
The dosage of citric acid : 6 mg/tablet

FIG. 1

EP 4 778 515 A1

**Description**

The present application claims priority to the prior Patent Application No. 202311186977.2 entitled "RISPERIDONE SOLID ORAL FILM, PREPARATION METHOD THEREFOR AND USE THEREOF" and filed with the China National Intellectual Property Administration on September 14, 2023.

**TECHNICAL FIELD**

The present disclosure relates to a risperidone solid oral film administered via the gastrointestinal tract, a preparation method therefor, and use thereof.

**BACKGROUND**

Risperidone is a 5-hydroxytryptamine/dopamine antagonist with a unique balance mechanism. It is effective for positive and negative symptoms of schizophrenia, and can also improve the cognitive function and facilitate the re-entry of patients into society. Risperidone, as an atypical novel antipsychotic, can selectively inhibit the mesolimbic dopaminergic nervous system, without producing severe extrapyramidal responses as traditional anti-schizophrenia drugs do. It has few adverse effects during clinical administration, high safety, and relatively low price, and is one of the first choice drugs for resisting schizophrenia at present.

At present, risperidone formulations for clinical use in China mainly include tablets (1 mg, 2 mg, and 3 mg), dispersible tablets (1 mg), capsules (1 mg), oral liquid (30 mL:30 mg), orally disintegrating tablets (0.5 mg, 1 mg, and 2 mg), microsphere injectable liquid (25 mg, 37.5 mg, and 50 mg), and the like. Risperidone tablets with the trade name "Weisitong" manufactured by Xi'an Janssen Pharmaceutical Co., Ltd. were the first marketed product in China and were approved for marketing in 2001.

Common tablets and capsules need to be taken with water, which is inconvenient especially for children and elderly patients with dysphagia, and patients with mental disorders often refuse to take the medicine or spit the medicine. Although the orally disintegrating tablets solve the above problems to a certain extent, the orally disintegrating tablets have an unpleasant sandy feel when taken. In addition, the orally disintegrating tablets are fragile during transportation, which easily causes inaccurate administration dosage and higher requirements for production, packaging, and preservation. Special measuring cups are needed when oral liquids are taken, which is inconvenient to carry and affects the wide application thereof in clinic. According to the dosage and administration in the specification of this product, the minimum dose of this product is 0.25 mg. However, there is no low dose of 0.25 mg for oral solid drugs on the market at present.

In order to improve medication compliance in patients with mental disorders, patent document CN101632651A discloses a risperidone oral fast-soluble film, the components of which comprise risperidone, a water-soluble pharmaceutical high-molecule excipient, an additive (a flavoring agent, a colorant, an opacifier, a plasticizer, a preservative, a pH regulator, and a disintegrant), and water. However, the invention ignores one property of the risperidone starting material itself, that is, the risperidone starting material has a relatively strong bitter taste. Although the formula contains a flavoring agent, it is not enough to mask the strong bitter taste of risperidone, and the drug will be in direct contact with oral taste buds for a period of time during administration, so the bitter taste will greatly affect the compliance of patients with mental disorders during administration, thereby affecting the clinical use thereof to a certain extent.

Patent document CN103349657A discloses a risperidone film formulation. In order to mask the bitter taste of risperidone itself, a multiple-strip composite film is invented, including an acidic strip film strip and a basic strip film strip. When taken, the composite film forms an effervescent film when contacting water, resulting in a large number of bubbles, which paralyze the sense of smell and thus mask the taste. However, in the production of the invention, a multiple-strip film strip needs to be prepared, the production process is complex, the cost is relatively high, and it is not suitable for industrial production.

Patent document CN108685876A discloses an oral film pharmaceutical composition comprising risperidone. Risperidone has low solubility in water, is present in particulate form, and is bitter. According to the invention, after risperidone is dissolved by adding an acidic substance, povidone is then added for taste masking. However, the product prepared by the method has poor stability.

Patent document CN104546806A discloses an oral fast-soluble film comprising risperidone and a preparation method therefor. According to the invention, the taste masking effect is achieved by cyclodextrin inclusion, and the excipient includes cyclodextrin, a film-forming material, a plasticizer, and a flavoring agent. In the preparation process, a risperidone clathrate is prepared by a saturated aqueous solution-ultrasonic cell pulverization method. The cyclodextrin and risperidone solutions are mixed, subjected to ultrasonication for 100 times, refrigerated in a refrigerator at 4 °C for 24 h, filtered under reduced pressure, dried at 40 °C for 4 h to constant weight, and ground. Such preparation process is complicated, has low efficiency and automation degree, and is not easy for industrial production. In addition, the inclusion rate is not explicitly controlled in the invention, and there is no step for removing free drugs. If the inclusion rate is low and there is risperidone that is not included, the prepared oral fast-soluble film will still have the problem of bitter taste.

Patent document CN113842376A discloses an oral fast-soluble film comprising risperidone and a preparation method therefor. The drug substance and Eudragit are used as a coating solution and fluidizedly coated on the surface of mannitol, and then the Eudragit coating solution is further coated to obtain an intermediate containing the drug substance. In this coating mode, a compact coating film can be formed to encapsulate risperidone therein, which can completely mask the bitter taste of risperidone and is more acceptable to the patient. In addition, after coating, the contact of risperidone with oxygen and some excipients incompatible with risperidone can be isolated, the generation of oxidative impurities and other impurities can be effectively reduced, and the stability of the formulation is excellent. However, the process is complex and difficult to operate, which is not conducive to production. Moreover, Eudragit is expensive and has relatively high costs.

Patent document CN107028917B is intended to increase compliance, thereby preparing an oral soluble film. However, the addition of high-concentration ethanol as a solvent has relatively high production requirements, poses certain risks during operation, and causes pollution to the environment.

In conclusion, there is an urgent need to develop a new dosage form comprising risperidone which has good mouthfeel, has wide dose coverage, is easy to accept and use by patients, has good stability, has fast dissolution, has low production cost, and/or is suitable for industrial production.

## SUMMARY

The present disclosure provides a risperidone solid oral film, wherein the risperidone solid oral film in each dosage unit comprises: 0.10%-8.00% of risperidone, 5.00%-40.00% of a flavoring agent, 5.00%-40.00% of a stabilizer, 2.00%-15.00% of a plasticizer, 0%-45.00% of a filler, 2.00%-8.00% of a colorant, and 25.00%-70.00% of a film-forming material; the percentage refers to the percentage of the mass of each of the components relative to the total mass of the risperidone solid oral film; the risperidone solid oral film does not comprise polyvinylpyrrolidone and a coating material.

According to an embodiment of the present disclosure, the risperidone solid oral film in each dosage unit comprises 0.1 mg-3 mg of risperidone, such as 0.25 mg, 1 mg, or 2 mg of risperidone. According to an embodiment of the present disclosure, the coating material refers to a conventional coating material in the art, such as Eudragit.

According to an embodiment of the present disclosure, the content of the risperidone is preferably 0.10%-8.00%, further preferably 0.40%-7.00%, such as 6.50%, 6.00%, 5.56%, 5.41%, 5.13%, 5.00%, 4.88%, 4.51%, 4.44%, 4.29%, 4.00%, 3.50%, 3.00%, 2.50%, 2.01%, 1.50%, 1.00%, 0.56%, 0.54%, 0.50%, or 0.45%; the percentage refers to the percentage of the mass of risperidone relative to the total mass of the risperidone solid oral film.

According to an embodiment of the present disclosure, the flavoring agent is a substance that has a flavoring effect in the film and is selected from one or more of sucrose, mannitol, dextran, sucralose, aspartame, menthol, and sodium chloride.

In some embodiments, the flavoring agent is selected from one or more of sucralose, menthol, and sodium chloride.

In one embodiment, the flavoring agent consists of sucralose, menthol, and sodium chloride, or consists of sucralose and sodium chloride.

According to an embodiment of the present disclosure, the content of the flavoring agent is preferably 5.00%-40.00%, further preferably 6.00%-35.00%, such as 32.00%, 30.00%, 27.02%, 25.64%, 25.00%, 24.99%, 24.39%, 22.54%, 22.23%, 21.62%, 21.46%, 20.00%, 15.00%, 10.00%, 8.03%, 7.50%, 7.00%, or 6.50%; the percentage refers to the percentage of the mass of the flavoring agent relative to the total mass of the risperidone solid oral film.

According to an embodiment of the present disclosure, the stabilizer refers to a substance that keeps the finished product stable and is selected from one or more of citric acid (e.g., anhydrous citric acid or citric acid monohydrate) and/or sodium citrate, tartaric acid, hydrochloric acid, and malic acid. In some embodiments, the stabilizer is selected from citric acid.

According to an embodiment of the present disclosure, the content of the stabilizer is preferably 5.00%-40.00%, further preferably 5.00%-35.00%, such as 5.41%, 5.56%, 6.63%, 9.86%, 10.26%, 10.81%, 14.16%, 14.63%, 15.00%, 17.50%, 20.00%, 22.22%, 25.00%, 30.00%, or 32.00%; the percentage refers to the percentage of the mass of the stabilizer relative to the total mass of the risperidone solid oral film.

According to an embodiment of the present disclosure, the plasticizer refers to a substance for reducing the glass transition temperature of the film, increasing the plasticity and toughness, and improving the stretching rate, and is selected from one or more of glycerol, propylene glycol, sorbitol, and polyethylene glycol. In some embodiments, the plasticizer is glycerol.

According to an embodiment of the present disclosure, the content of the plasticizer is preferably 2.00%-15.00%, further preferably 2.00%-13.00%, such as 2.70%, 3.50%, 4.00%, 4.29%, 4.44%, 4.51%, 4.88%, 5.13%, 5.41%, 5.56%, 6.00%, 7.00%, 8.00%, 9.00%, 10.04%, 11.00%, or 12.00%; the percentage refers to the percentage of the mass of the plasticizer relative to the total mass of the risperidone solid oral film.

According to an embodiment of the present disclosure, the filler refers to a solid substance that is added to a material to improve the properties of the material, or to solubilize and increase weight, and to reduce the cost of the material, and is selected from one or more of microcrystalline cellulose (MCC), mannitol, starch, pregelatinized starch, maltodextrin, sucrose, lactose, sorbitol, and glucose. In some embodiments, the filler is microcrystalline cellulose.

According to an embodiment of the present disclosure, the content of the filler is preferably 0-45.00%, such as 43.00%, 42.00%, 41.00%, 40.16%, 35.00%, 30.00%, 25.00%, 20.00%, 15.21%, 15.00%, 14.48%, 11.27%, 10.73%, 10.00%, 5.00%, or 0; the percentage refers to the percentage of the mass of the filler relative to the total mass of the risperidone solid oral film.

According to an embodiment of the present disclosure, the colorant refers to a substance that is capable of improving the appearance and color of a formulation and can be used for identifying the concentration of the formulation, distinguishing the application method, and reducing the patient's aversion to the administration, and is selected from titanium dioxide.

According to an embodiment of the present disclosure, the content of the colorant is preferably 2.00%-8.00%, further preferably 2.00%-7.00%, such as 2.00%, 2.15%, 2.23%, 2.25%, 2.44%, 2.56%, 2.70%, 2.77%, 3.01%, 3.50%, 4.00%, 4.50%, 5.00%, 5.41%, 5.50%, 6.00%, or 6.50%; the percentage refers to the percentage of the mass of the colorant relative to the total mass of the risperidone solid oral film.

According to an embodiment of the present disclosure, the film-forming material is a carrier of a drug and is selected from hydroxypropyl methylcellulose (HMPC, e.g., hydroxypropyl methylcellulose E15) and/or polyvinyl alcohol.

According to an embodiment of the present disclosure, the content of the film-forming material is preferably 25.00%-70.00% or 25.00%-65.00%, such as 60.00%, 55.56%, 55.00%, 54.05%, 51.28%, 50.00%, 48.78%, 45.07%, 44.44%, 42.92%, 40.00%, 35.00%, 30.12%, 28.00%, or 26.00%; the percentage refers to the percentage of the mass of the film-forming material relative to the total mass of the risperidone solid oral film.

According to one embodiment of the present disclosure, for the risperidone solid oral film, the oral film in each dosage unit comprises or consists essentially of the following components:

1.00%-3.00% of risperidone, 5.00%-10.00% of a flavoring agent, 5.00%-10.00% of a stabilizer, 7.00%-13.00% of a plasticizer, 35.00%-45.00% of a filler, 2.00%-5.00% of a colorant, and 25.00%-40.00% of a film-forming material; the percentage refers to the percentage of the mass of each of the components relative to the total mass of the risperidone solid oral film; the risperidone solid oral film does not comprise polyvinylpyrrolidone and a coating material; preferably, the flavoring agent consists of sucralose and sodium chloride; for example, sucralose and sodium chloride are in a mass ratio of 1:(1-3), such as 1:1, 1:1.5, 1:1.6, 1:1.7, 1:2, or 1:2.5; the stabilizer is citric acid, the plasticizer is glycerol, the filler is microcrystalline cellulose, the colorant is titanium dioxide, and the film-forming material is hydroxypropyl methylcellulose. According to an embodiment of the present disclosure, the risperidone solid oral film described herein may be any one of the following formulas:

formula 1: 5.56% risperidone, 8.33% sucralose, 2.77% menthol, 13.89% sodium chloride, 5.56% anhydrous citric acid, 5.56% glycerol, 2.77% titanium dioxide, and 55.56% hydroxypropyl methylcellulose, wherein the percentage refers to the percentage of the mass of each of the components relative to the total mass of the risperidone solid oral film;

formula 2: 5.41% risperidone, 8.11% sucralose, 13.51% sodium chloride, 10.81% anhydrous citric acid, 5.41% glycerol, 2.70% titanium dioxide, and 54.05% hydroxypropyl methylcellulose, wherein the percentage refers to the percentage of the mass of each of the components relative to the total mass of the risperidone solid oral film;

formula 3: 5.41% risperidone, 8.10% sucralose, 5.41% menthol, 13.51% sodium chloride, 5.41% anhydrous citric acid, 2.70% glycerol, 5.41% titanium dioxide, and 54.05% hydroxypropyl methylcellulose, wherein the percentage refers to the percentage of the mass of each of the components relative to the total mass of the risperidone solid oral film;

formula 4: 5.41% risperidone, 8.10% sucralose, 5.41% menthol, 13.51% sodium chloride, 5.41% anhydrous citric acid, 2.70% glycerol, 5.41% titanium dioxide, and 54.05% polyvinyl alcohol, wherein the percentage refers to the percentage of the mass of each of the components relative to the total mass of the risperidone solid oral film;

formula 5: 5.41% risperidone, 8.10% sucralose, 5.41% menthol, 13.51% sodium chloride, 5.41% citric acid, 5.41% glycerol, 2.70% titanium dioxide, and 54.05% hydroxypropyl methylcellulose, wherein the percentage refers to the percentage of the mass of each of the components relative to the total mass of the risperidone solid oral film;

formula 6: 5.13% risperidone, 7.69% sucralose, 5.13% menthol, 12.82% sodium chloride, 10.26% citric acid, 5.13% glycerol, 2.56% titanium dioxide, and 51.28% hydroxypropyl methylcellulose, wherein the percentage refers to the percentage of the mass of each of the components relative to the total mass of the risperidone solid oral film;

formula 7: 4.88% risperidone, 7.32% sucralose, 4.88% menthol, 12.19% sodium chloride, 14.63% citric acid, 4.88% glycerol, 2.44% titanium dioxide, and 48.78% hydroxypropyl methylcellulose, wherein the percentage refers to the percentage of the mass of each of the components relative to the total mass of the risperidone solid oral film;

formula 8: 4.44% risperidone, 6.67% sucralose, 4.44% menthol, 11.12% sodium chloride, 22.22% citric acid, 4.44% glycerol, 2.23% titanium dioxide, and 44.44% hydroxypropyl methylcellulose, wherein the percentage refers to the percentage of the mass of each of the components relative to the total mass of the risperidone solid oral film;

formula 9: 4.00% risperidone, 6.00% sucralose, 4.00% menthol, 10.00% sodium chloride, 30.00% anhydrous citric acid, 4.00% glycerol, 2.00% titanium dioxide, and 40.00% hydroxypropyl methylcellulose, wherein the percentage refers to the percentage of the mass of each of the components relative to the total mass of the risperidone solid oral film;

formulas 10-12: 2.01% risperidone, 3.01% sucralose, 40.16% microcrystalline cellulose, 5.02% sodium chloride, 6.63% citric acid monohydrate, 10.04% glycerol, 3.01% titanium dioxide, and 30.12% hydroxypropyl methylcellulose, wherein the percentage refers to the percentage of the mass of each of the components relative to the total mass of the risperidone solid oral film;

formula 13: 0.56% risperidone, 6.76% sucralose, 4.51% menthol, 11.27% sodium chloride, 9.86% citric acid monohydrate, 4.51% glycerol, 15.21% microcrystalline cellulose, 2.25% titanium dioxide, and 45.07% hydroxypropyl methylcellulose, wherein the percentage refers to the percentage of the mass of each of the components relative to the total mass of the risperidone solid oral film;

formula 14: 4.51% risperidone, 6.76% sucralose, 4.51% menthol, 11.27% sodium chloride, 9.86% citric acid monohydrate, 4.51% glycerol, 11.26% microcrystalline cellulose, 2.25% titanium dioxide, and 45.07% hydroxypropyl methylcellulose, wherein the percentage refers to the percentage of the mass of each of the components relative to the total mass of the risperidone solid oral film;

formula 15: 0.54% risperidone, 6.44% sucralose, 4.29% menthol, 10.73% sodium chloride, 14.16% citric acid monohydrate, 4.29% glycerol, 14.48% microcrystalline cellulose, 2.15% titanium dioxide, and 42.92% hydroxypropyl methylcellulose, wherein the percentage refers to the percentage of the mass of each of the components relative to the total mass of the risperidone solid oral film;

formula 16: 4.29% risperidone, 6.44% sucralose, 4.29% menthol, 10.73% sodium chloride, 14.16% citric acid monohydrate, 4.29% glycerol, 10.73% microcrystalline cellulose, 2.15% titanium dioxide, and 42.92% hydroxypropyl methylcellulose, wherein the percentage refers to the percentage of the mass of each of the components relative to the total mass of the risperidone solid oral film.

The present disclosure further provides a preparation method for the risperidone solid oral film, which comprises the following steps:

step 1: mixing two, more or all of a solvent, a flavoring agent, a stabilizer, a plasticizer, a colorant, a filler, and a film-forming material according to a formula amount, performing electrically stirring or vacuum-emulsifying until uniform, and performing vacuum deaeration to obtain a drug-containing gel solution;

step 2: adding the drug-containing gel solution obtained in step 1 into a film preparation machine for film coating and drying to obtain a dry film intermediate;

step 3: cutting and packaging the dry film intermediate obtained in step 2 to obtain the risperidone solid oral film.

According to an embodiment of the present disclosure, in step 2, the temperature of the drying is preferably 60 ±10 °C, and the time of the drying is preferably 20 min.

According to an embodiment of the present disclosure, the risperidone solid oral film may be a risperidone fast-soluble and/or immediate-release solid oral film administered via the gastrointestinal tract.

According to an embodiment of the present disclosure, the risperidone solid oral film has a dry film thickness of 10 μm=200 μm, such as 30 μm, 40 μm, 50 μm, 60 μm, 70 μm, 80 μm, 90 μm, or 100 μm.

The present disclosure further provides use of the risperidone solid oral film for manufacturing a medicament for treating and/or preventing a neuropsychiatric disease and/or a psychiatric disorder.

According to an embodiment of the present disclosure, the neuropsychiatric disease and the psychiatric disorder are selected from the group consisting of schizophrenia, psychotic disorder, Alzheimer's disease, frontotemporal dementia, vascular dementia, dementia with Lewy bodies, senile dementia, mild cognitive impairment, benign amnesia, closed head trauma, autism spectrum disorder, Asperger's disease, fragile X syndrome, attention deficit hyperactivity disorder, attention deficit disorder, obsessive compulsive disorder, tic disorder, learning disorder in children, premenstrual syndrome, depression, major depression, anhedonia, suicidal ideation and/or behavior, bipolar disorder, anxiety disorder, panic disorder, post-traumatic stress disorder, chronic mild and unpredictable stress, eating disorder, addiction disorder, personality disorder, Parkinson's disease, Huntington's disorder, multiple sclerosis, amyotrophic lateral sclerosis, ataxia, Friedreich's ataxia, Tourette's syndrome, nocturnal enuresis, non-epileptic seizure, blepharospasm, Duchenne muscular dystrophy, stroke, chronic pain, neuropathic pain, hyperalgesia, allodynia, diabetic polyneuropathy, epileptic seizure, and epilepsy.

The present disclosure further provides use of the risperidone solid oral film for manufacturing a pharmaceutical formulation.

According to an embodiment of the present disclosure, the pharmaceutical formulation is a pharmaceutical formulation for treating and/or preventing a neuropsychiatric disease and/or a psychiatric disorder. Preferably, the neuropsychiatric disease and the psychiatric disorder are defined as described above.

According to an embodiment of the present disclosure, the pharmaceutical formulation may be a risperidone fast-soluble and/or immediate-release solid oral film administered via the gastrointestinal tract.

The present disclosure further provides a method for treating and/or preventing a neuropsychiatric disease and/or a psychiatric disorder, comprising providing a therapeutically effective amount of the risperidone solid oral film to a patient in need.

According to an embodiment of the present disclosure, the neuropsychiatric disease and the psychiatric disorder have the meanings as indicated above.

In one embodiment, the neuropsychiatric disease and/or the psychiatric disorder is schizophrenia. The reagents and starting materials used in the present disclosure are commercially available.

Beneficial Effects of Present Disclosure:

The inventors have found that risperidone belongs to a low-solubility and high-permeability drug, and its low solubility and slow dissolution in the gastrointestinal tract limit the absorption of the drug; the strong bitter taste of risperidone is not easily masked, and the oral drug compliance of patients is poor. The risperidone solid oral film administered via the gastrointestinal tract provided herein has at least one of the following advantages:

firstly, the oral film is fast-soluble and/or immediate-release, is dissolved relatively fast, is completely dissolved in four dissolution media of water, a 0.1 mol/L hydrochloric acid solution, an acetic acid-sodium acetate buffer (pH 4.5), and a phosphate buffer (pH 6.8) within 10 min, and has a relatively high dissolution rate;

secondly, the oral film has good mouthfeel and good patient compliance;

thirdly, the oral film has good stability, such as good stability under high temperature, high humidity, light-shielding, acceleration, and long-term conditions;

fourthly, the preparation method therefor is simple to operate, has good reproducibility, and is suitable for industrial production.

Definitions and Description

The term "oral film in each dosage unit" refers to a unit dose of an oral film, i.e., an oral film containing the above percentages of risperidone, flavoring agent, stabilizer, plasticizer, filler, colorant, and film-forming material, is loaded into 1 unit package.

The term "more" refers to two or more, e.g., two, three or more.

The term "consisting essentially of" means that the sum of the mass of these components is no less than 85%, e.g., no less than 90%, illustratively 90%, 91%, 92%, 93%, 94%, 95%, 96%, 97%, 98%, 99%, or 100% of the total mass of the risperidone solid oral film.

The term "patient" refers to any animal including mammals, preferably mice, rats, other rodents, rabbits, dogs, cats, pigs, cattle, sheep, horses, or primates, and most preferably humans.

The term "therapeutically effective amount" refers to the amount of the active compound or drug that causes a biological or medical response that researchers, veterinarians, physicians, or other clinicians are looking for in tissues, systems, animals, individuals, or humans.

**BRIEF DESCRIPTION OF THE DRAWINGS**

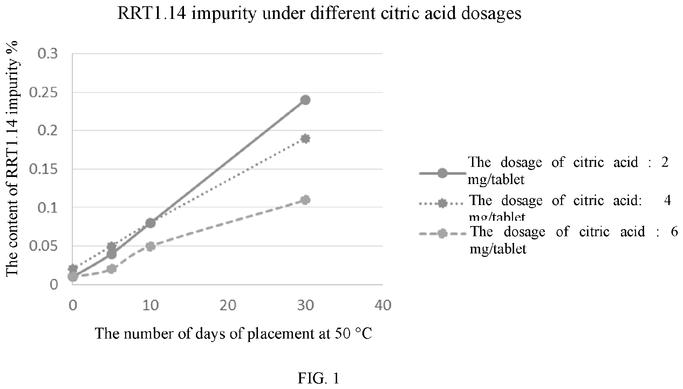

FIG. 1 shows the main impurities of formulas 5-7 in Example 3,

wherein ━━●━━ represents an impurity content line when citric acid is used at an amount of 2 mg/g tablet at 50 °C;

• • • ● • • • represents an impurity content line when citric acid is used at an amount of 4 mg/g tablet at 50 °C;

━ ━ ● ━ ━ represents an impurity content line when citric acid is used at an amount of 6 mg/g tablet at 50 °C.

DETAILED DESCRIPTION

The technical solutions of the present disclosure are further described in detail below with reference to specific examples. It will be appreciated that the following examples are merely exemplary illustrations and explanations of the present disclosure and should not be construed as limiting the claimed scope of the present disclosure. All techniques implemented on the basis of the content described above of the present disclosure fall within the claimed scope of the present disclosure.

Unless otherwise stated, the starting materials and reagents used in the following examples are all commercially available products or can be prepared by using known methods or can be selected according to the commodity instructions.

The API content assay method is as follows:
determination was performed according to the high performance liquid chromatography (General Chapter 0512, Chinese Pharmacopoeia, Volume IV, 2020 Edition).

Chromatographic conditions: octadecylsilane bonded silica gel was used as a filler (RD-C 18 of Zhongpu Technology, 4.6 mm × 150 mm, 5 $\mu$m, or a chromatographic column with equivalent performance is recommended); ammonium acetate buffer (5 g of ammonium acetate was dissolved in 1000 mL of water)-methanol (40:60) was a mobile phase; the flow rate was 1.0 mL/min; the column temperature was 30 °C; the detection wavelength was 275 nm; the injection volume was 10 $\mu$L; and the running time was 10 minutes.

The method for detecting the related substances is as follows:
determination was performed according to the high performance liquid chromatography (General Chapter 0512, Chinese Pharmacopoeia, Volume IV, 2020 Edition).

Chromatographic conditions: octadecylsilane bonded silica gel was used as a filler (Waters XSelect HSS C18, 4.6 mm × 250 mm, 3.5 $\mu$m, or a chromatographic column with equivalent performance is recommended); ammonium acetate buffer (5 g of ammonium acetate was dissolved in 1000 mL of water)-acetonitrile (70:30) was mobile phase A; methanol was mobile phase B; gradient elution was performed according to the following table; the detection wavelength was 260 nm; the flow rate was 1.0 mL/min; the column temperature was 30 °C; and the injection volume was 30 $\mu$L.

| Time (min) | Mobile phase A (%) | Mobile phase B (%) |
|---|---|---|
| 0 | 100 | 0 |
| 50 | 50 | 50 |
| 55 | 50 | 50 |
| 56 | 100 | 0 |
| 65 | 100 | 0 |

Example 1

Table 1. Formula summary table of Example 1

| Formula mg | | Formula 1 | Percentage % | Formula 2 | Percentage % |
|---|---|---|---|---|---|
| Solvent | Purified water* | q.s. | / | q.s. | / |
| Flavoring agent | Sucralose | 3 | 8.33 | 3 | 8.11 |
| Flavoring agent | Menthol | 1 | 2.77 | / | / |
| Flavoring agent | Sodium chloride | 5 | 13.89 | 5 | 13.51 |
| Stabilizer | Anhydrous citric acid | 2 | 5.56 | 4 | 10.81 |
| Plasticizer | Glycerol | 2 | 5.56 | 2 | 5.41 |
| Colorant | Titanium dioxide | 1 | 2.77 | 1 | 2.70 |

(continued)

| Formula mg | | Formula 1 | Percentage % | Formula 2 | Percentage % |
|---|---|---|---|---|---|
| Drug sub-stance | Risperidone | 2 | 5.56 | 2 | 5.41 |
| Film-form-ing materi-al | Hydroxypropyl methylcellulose | 20 | 55.56 | 20 | 54.05 |
| Tablet weight | | 36 | 100 | 37 | 100 |
| Mouthfeel | | After tasting, the mouthfeel was sour and sweet, there was no bitter taste, and the taste was cold. | | After tasting, the mouthfeel was sour and sweet, with no bitter taste. | |
| *Purified water was removed after film preparation and drying. | | | | | |

Preparation method of formula 1 and formula 2:

step 1: a solvent, a flavoring agent, a stabilizer, a plasticizer, a colorant, a drug substance, and a film-forming material were added according to the formula amount shown in Table 1, electrically stirring or vacuum-emulsifying was performed until uniform, and vacuum deaeration was performed to obtain a drug-containing gel solution;
step 2: the drug-containing gel solution obtained in step 1 was added into a film preparation machine for film coating and drying at a drying parameter of $60 \pm 10$ °C/20 min to obtain a dry film intermediate;
step 3: the dry film intermediate obtained in step 2 was cut and packaged to obtain the risperidone solid oral film.

Example 2

The formula table is as follows:

Table 2. Formula summary table of Example 2

| Formula mg | | Formula 3 | Percentage % | Formula 4 | Percentage % |
|---|---|---|---|---|---|
| Solvent | Purified water* | q.s. | / | q.s. | / |
| Flavoring agent | Sucralose | 3 | 8.10 | 3 | 8.10 |
| Flavoring agent | Menthol | 2 | 5.41 | 2 | 5.41 |
| Flavoring agent | Sodium chloride | 5 | 13.51 | 5 | 13.51 |
| Stabilizer | Anhydrous citric acid | 2 | 5.41 | 2 | 5.41 |
| Plasticizer | Glycerol | 1 | 2.70 | 1 | 2.70 |
| Colorant | Titanium dioxide | 2 | 5.41 | 2 | 5.41 |
| Drug substance | Risperidone | 2 | 5.41 | 2 | 5.41 |
| Film-forming material | Hydroxypropyl methylcellulose | 20 | 54.05 | / | / |
| | Polyvinyl alcohol | / | / | 20 | 54.05 |
| Tablet weight | | 37 | 100 | 37 | 100 |
| *Purified water was removed after film preparation and drying. | | | | | |

Preparation method of formulas 3 and 4:

step 1: a solvent, a flavoring agent, a stabilizer, a plasticizer, a colorant, a drug substance, and a film-forming material were added according to the formula amount shown in Table 2, electrically stirring or vacuum-emulsifying was performed until uniform, and vacuum deaeration was performed to obtain a drug-containing gel solution;
step 2: the drug-containing gel solution obtained in step 1 was added into a film preparation machine for film coating and drying at a drying parameter of $60 \pm 10$ °C/20 min to obtain a dry film intermediate;
step 3: the dry film intermediate obtained in step 2 was cut and packaged to obtain the risperidone solid oral film.

The obtained oral film samples were placed at a high temperature of 50 °C for stability testing and taken out on days 5, 10, and 30, and the content of related substances was investigated. Summary data are shown in Table 3:

Table 3. Formula stability summary table of Example 2

| batch No. | Formula 3 | | | | Formula 4 | | | |
|---|---|---|---|---|---|---|---|---|
| Storage conditions | 0 days | 50 °C, 5 days | 50 °C, 10 days | 50 °C, 30 days | 0 days | 50 °C, 5 days | 50 °C, 10 days | 50 °C, 30 days |
| RRT (relative retention time) | % Area | | | | % Area | | | |
| 0.08 | / | / | / | / | / | / | 0.01 | / |
| 0.08 | / | / | / | / | / | / | 0.01 | / |
| 0.54 | / | / | / | / | / | / | / | 0.02 |
| IM-Bicyclo(0.59) | / | / | / | / | / | / | / | 0.01 |
| 0.64 | / | 0.01 | 0.02 | 0.01 | / | / | / | / |
| IM-cis-N-oxide(0.66) | 0.00 | 0.01 | 0.01 | 0.02 | 0.01 | 0.02 | 0.02 | 0.12 |
| IM-A(0.69) | / | / | / | / | / | / | / | 0.01 |
| 0.8 | / | / | / | 0.01 | / | / | / | 0.04 |
| IM-C(0.83) | / | / | / | / | / | 0.02 | 0.01 | 0.03 |
| 0.84 | / | 0.01 | 0.02 | 0.04 | / | 0.05 | 0.05 | 0.16 |
| 0.88 | / | / | / | / | / | / | / | / |
| 0.89 | / | / | / | 0.01 | / | / | / | 0.02 |
| 0.92 | / | / | / | / | / | / | / | 0.01 |
| IM-D(0.95) | / | 0.02 | 0.02 | 0.01 | / | 0.01 | 0.03 | 0.05 |
| 0.98 | 0.01 | 0.01 | 0.01 | 0.03 | / | 0.01 | 0.01 | 0.02 |
| API(1.00) | 99.98 | 99.83 | 99.74 | 99.56 | 99.97 | 99.83 | 99.74 | 98.86 |
| 1.04 | / | / | / | / | / | / | / | 0.07 |
| 1.06 | / | / | / | 0.02 | / | / | / | / |
| 1.09 | / | 0.03 | 0.03 | 0.03 | / | 0.02 | 0.05 | 0.1 |
| 1.14 | 0.01 | 0.04 | 0.08 | 0.24 | 0.02 | 0.02 | 0.05 | 0.39 |
| 1.17 | / | / | / | / | / | / | / | 0.01 |
| 1.25 | / | / | 0.02 | / | / | / | / | / |
| 1.31 | / | 0.02 | 0.04 | 0.01 | / | / | 0.02 | 0.08 |
| 1.39 | / | 0.01 | 0.02 | 0.03 | / | / | / | / |
| Total impurity | 0.02 | 0.16 | 0.27 | 0.46 | 0.03 | 0.15 | 0.26 | 1.14 |
| Note: "/" means undetectable. | | | | | | | | |

As can be seen from the 50 °C and 30-day data of formula 3 and formula 4, when the film-forming material was hydroxypropyl methylcellulose, the single impurities and total impurities were relatively small, and the stability was high.

Example 3

The formulas are summarized as follows:

Table 4 Formula summary table of Example 3

| Formula mg | | Formula 5 | Percentage % | Formula 6 | Percentage % | Formula 7 | Percentage % | Formula 8 | Percentage % | Formula 9 | Percentage % |
|---|---|---|---|---|---|---|---|---|---|---|---|
| Solvent | Purified water* | q.s. | / | q. s. | / | q.s. | / | q.s. | / | q.s. | / |
| Flavoring agent | Sucralose | 3 | 8.10 | 3 | 7.69 | 3 | 7.32 | 3 | 6.67 | 3 | 6.00 |
| Flavoring agent | Menthol | 2 | 5.41 | 2 | 5.13 | 2 | 4.88 | 2 | 4.44 | 2 | 4.00 |
| Flavoring agent | Sodium chloride | 5 | 13.51 | 5 | 12.82 | 5 | 12.19 | 5 | 11.12 | 5 | 10.00 |
| Stabilizer | Citric acid | 2 | 5.41 | 4 | 10.26 | 6 | 14.63 | 10 | 22.22 | 15 | 30.00 |
| Colorant | Titanium dioxide | 1 | 2.70 | 1 | 2.56 | 1 | 2.44 | 1 | 2.23 | 1 | 2.00 |
| Plasticizer | Glycerol | 2 | 5.41 | 2 | 5.13 | 2 | 4.88 | 2 | 4.44 | 2 | 4.00 |
| Drug substance | Risperidone | 2 | 5.41 | 2 | 5.13 | 2 | 4.88 | 2 | 4.44 | 2 | 4.00 |
| Film-forming material | Hydroxypropyl methylcellulose | 20 | 54.05 | 20 | 51.28 | 20 | 48.78 | 20 | 44.44 | 20 | 40.00 |
| Tablet weight | | 37 | 100 | 39 | 100 | 41 | 100 | 45 | 100 | 50 | 100 |
| Dry film condition | | No abnormality | | No abnormality | | No abnormality | | Water absorption, tendency to shrink | | Heavy water absorption | |
| *Purified water was removed after film preparation and drying. | | | | | | | | | | | |

Preparation method of formulas 5-9:

step 1: a solvent, a flavoring agent, a stabilizer, a plasticizer, a colorant, a drug substance, and a film-forming material were added according to the formula amount shown in Table 4, electrically stirring or vacuum-emulsifying was performed until uniform, and vacuum deaeration was performed to obtain a drug-containing gel solution;
step 2: the drug-containing gel solution obtained in step 1 was added into a film preparation machine for film coating and drying at a drying parameter of 60±10 °C/20 min to obtain a dry film intermediate;
step 3: the dry film intermediate obtained in step 2 was cut and packaged to obtain the risperidone solid oral film.

The oral film samples obtained by formulas 5-7 were placed at a high temperature of 50 °C for stability testing and taken out on days 5, 10, and 30, and the content of related substances was investigated. Summary data are shown in Table 5:

Table 5. Stability data summary table of formulas 5-7

| batch No. | Formula 5 | | | | Formula 6 | | | | Formula 7 | | | |
|---|---|---|---|---|---|---|---|---|---|---|---|---|
| Storage conditions | 0 days | 50 °C, 5 days | 50 °C, 10 days | 50 °C, 30 days | 0 days | 50 °C, 5 days | 50 °C, 10 days | 50 °C, 30 days | 0 days | 50 °C, 5 days | 50 °C, 10 days | 50 °C, 30 days |
| RRT (relative retention time) | % Area | | | | % Area | | | | % Area | | | |
| 0.54 | / | / | / | / | / | / | / | 0 | / | / | / | 0.01 |
| 0.56 | / | / | / | / | / | / | / | 0.01 | / | / | / | / |
| 0.6 | / | / | / | / | / | / | / | / | / | / | / | 0.01 |
| 0.64 | / | 0.01 | 0.02 | 0.01 | / | 0.01 | 0.02 | 0.01 | / | 0.01 | 0.02 | 0.02 |
| IM-cis-N-oxide(0.66) | 0 | 0.01 | 0.01 | 0.02 | 0.01 | 0.01 | 0.01 | 0.03 | 0.01 | 0.01 | 0.01 | 0.03 |
| 0.8 | / | / | / | 0.01 | / | / | / | 0.01 | / | / | / | / |
| 0.84 | / | 0.01 | 0.02 | 0.04 | / | 0.01 | 0.02 | 0.02 | / | / | 0.01 | 0.01 |
| 0.88 | / | / | / | / | / | / | / | 0 | / | / | / | / |
| 0.89 | / | / | / | 0.01 | / | / | / | 0.01 | / | / | / | / |
| IM-D(0.95) | / | 0.02 | 0.02 | 0.01 | / | / | / | / | / | / | / | / |
| 0.98 | 0.01 | 0.01 | 0.01 | 0.03 | / | 0.01 | 0.01 | 0.02 | / | / | / | / |
| API(1.00) | 99.98 | 99.83 | 99.74 | 99.56 | 99.98 | 99.87 | 99.82 | 99.58 | 99.98 | 99.91 | 99.75 | 99.61 |
| 1.04 | / | / | / | / | / | / | / | / | / | / | 0.05 | 0.08 |
| 1.06 | / | / | / | 0.02 | / | / | / | 0.03 | / | / | 0.03 | 0.03 |
| 1.09 | / | 0.03 | 0.03 | 0.03 | / | 0.02 | 0.02 | 0.03 | / | 0.02 | 0.02 | 0.01 |
| 1.14 | 0.01 | 0.04 | 0.08 | 0.24 | 0.02 | 0.05 | 0.08 | 0.19 | 0.01 | 0.02 | 0.05 | 0.11 |
| 1.15(IM-E) | / | / | / | / | / | / | / | / | / | / | / | 0.01 |
| 1.25 | / | / | 0.02 | / | / | / | / | / | / | / | / | / |
| 1.29 | / | / | / | / | / | / | / | / | / | 0.01 | 0.02 | 0.04 |
| 1.31 | / | 0.02 | 0.04 | 0.01 | / | 0.01 | 0.01 | 0.05 | / | 0.02 | 0.02 | / |
| 1.33 | / | / | / | / | / | / | / | / | / | / | / | 0.01 |
| 1.39 | / | 0.01 | 0.02 | 0.03 | / | 0.01 | 0.02 | 0.02 | / | 0.01 | 0.02 | 0.02 |
| Total impurity | 0.02 | 0.16 | 0.27 | 0.46 | 0.03 | 0.13 | 0.19 | 0.43 | 0.02 | 0.1 | 0.25 | 0.39 |

The content of the main impurity RRT1.14 is shown in FIG. 1.

Example 4

The formula table for risperidone solid oral films of 0.25 mg, 1 mg, and 2 mg specifications is as follows:

Table 6. Formulas 10-12

| Name mg | | Formula 10 | Formula 11 | Formula 12 | Percentage % |
|---|---|---|---|---|---|
| | | 0.25mg | 1mg | 2mg | |
| Solvent | Purified water* | q.s. | q.s. | q.s. | / |
| Flavoring agent | Sucralose | 0.375 | 1.5 | 3 | 3.01 |
| Flavoring agent | Sodium chloride | 0.625 | 2.5 | 5 | 5.02 |
| Stabilizer | Citric acid monohydrate** | 0.825 | 3.3 | 6.6 | 6.63 |
| Plasticizer | Glycerol | 1.25 | 5 | 10 | 10.04 |
| Drug substance | Risperidone (API) | 0.25 | 1 | 2 | 2.01 |
| Filler | Microcrystalline cellulose (MCC) | 5 | 20 | 40 | 40.16 |
| Colorant | Titanium dioxide | 0.375 | 1.5 | 3 | 3.01 |
| Film-forming material | Hydroxypropyl methylcellulose (HPMC) | 3.75 | 15 | 30 | 30.12 |
| Tablet weight | | 12.45 | 49.8 | 99.6 | 100% |

*Purified water was removed after film preparation and drying.
**Citric acid was calculated on an anhydrate basis. The content of anhydrous citric acid in the 2 mg specification risperidone solid oral film was 6 mg/tablet.

Preparation method of formulas 10-12:

step 1: a solvent, a flavoring agent, a stabilizer, a plasticizer, a drug substance, a colorant, a filler, and a film-forming material were added according to the formula amount shown in Table 6, electrically stirring or vacuum-emulsifying was performed until uniform, and vacuum deaeration was performed to obtain a drug-containing gel solution;
step 2: the drug-containing gel solution obtained in step 1 was added into a film preparation machine for film coating and drying at a drying parameter of 60±10 °C/20 min to obtain a dry film intermediate;
step 3: the dry film intermediate obtained in step 2 was cut and packaged to obtain the risperidone solid oral film.

Also, the dissolution of the risperidone solid oral films of formulas 10-12 in four dissolution media of water, a 0.1 mol/L hydrochloric acid solution, an acetic acid-sodium acetate buffer (pH 4.5), and a phosphate buffer (pH 6.8) was investigated. Dissolution test conditions were according to General Chapter 0931, Chinese Pharmacopoeia, Volume IV, 2020 Edition, Method 2: Paddle Method, with addition of settling basket: the volume of the dissolution medium was 500 mL, and the rotation speed was 50 rpm. The sampling time was 5, 10, 15, 30, and 60 min, and the samples were separately injected for determination by an HPLC method. The dissolution results are summarized in Table 7:

Table 7. Summary table of dissolution of formula 10 (specification: 0.25 mg) in 4 media

| Formula | 10 | | | | | | | |
|---|---|---|---|---|---|---|---|---|
| Medium | 0.1 M hydrochloric acid solution | | pH 4.5 Acetic acid-sodium acetate buffer | | pH 6.8 Phosphate buffer | | Water | |
| Time min | Cumulative dissolution rate % | RSD % | Cumulative dissolution rate % | RSD % | Cumulative dissolution rate % | RSD % | Cumulative dissolution rate % | RSD % |
| 5 | 107.4 | 2.9 | 96.4 | 9.6 | 103 | 2.6 | 102.2 | 2.6 |
| 10 | 108.2 | 2.7 | 94.1 | 8.6 | 105.5 | 2.2 | 102.6 | 2.6 |
| 15 | 107.7 | 3.0 | 96.1 | 10.1 | 105.5 | 2.2 | 102.8 | 2.0 |
| 30 | 107.4 | 2.9 | 96.2 | 7.1 | 105.4 | 2.2 | 103 | 1.9 |

(continued)

| Formula | 10 | | | | | | | |
|---|---|---|---|---|---|---|---|---|
| Medium | 0.1 M hydrochloric acid solution | | pH 4.5 Acetic acid-sodium acetate buffer | | pH 6.8 Phosphate buffer | | Water | |
| Time min | Cumulative dissolution rate % | RSD % | Cumulative dissolution rate % | RSD % | Cumulative dissolution rate % | RSD % | Cumulative dissolution rate % | RSD % |
| 60 | 107 | 3.2 | 95.3 | 5.1 | 104.3 | 2.6 | 103 | 2.0 |

Table 8. Summary table of dissolution of formula 11 (specification: 1 mg) in 4 media

| Formula | 11 | | | | | | | |
|---|---|---|---|---|---|---|---|---|
| Medium | 0.1 M hydrochloric acid solution | | pH 4.5 Acetic acid-sodium acetate buffer | | pH 6.8 Phosphate buffer | | Water | |
| Time min | Cumulative dissolution rate % | RSD% | Cumulative dissolution rate % | RSD% | Cumulative dissolution rate % | RSD% | Cumulative dissolution rate % | RSD% |
| 5 | 97.1 | 6.3 | 98.6 | 3.5 | 98.4 | 5.5 | 92.5 | 6.1 |
| 10 | 103.1 | 1.9 | 101.8 | 1.8 | 103 | 1.8 | 96 | 2.5 |
| 15 | 104.0 | 1.2 | 101.8 | 1.7 | 103.5 | 1.1 | 96.1 | 2.8 |
| 30 | 104.1 | 1.1 | 101.6 | 1.7 | 103.6 | 0.9 | 95.3 | 2.6 |
| 60 | 104.1 | 1.1 | 101.8 | 0.9 | 103.6 | 1.2 | 96.8 | 2.1 |

Table 9. Summary table of dissolution of formula 12 (specification: 2 mg) in 4 media

| Formula | 12 | | | | | | | |
|---|---|---|---|---|---|---|---|---|
| Medium | 0.1 M hydrochloric acid solution | | pH 4.5 Acetic acid-sodium acetate buffer | | pH 6.8 Phosphate buffer | | Water | |
| Time min | Cumulative dissolution rate % | RSD% | Cumulative dissolution rate % | RSD% | Cumulative dissolution rate % | RSD% | Cumulative dissolution rate % | RSD% |
| 5 | 91.6 | 8.7 | 89.2 | 7.8 | 82.1 | 8.7 | 76.9 | 19.2 |
| 10 | 102.3 | 4.0 | 100.6 | 2.9 | 98.9 | 1.8 | 90.1 | 12.1 |
| 15 | 103.7 | 2.9 | 102.5 | 1.3 | 102.0 | 0.8 | 94.5 | 6.9 |
| 30 | 104.2 | 1.1 | 102.0 | 1.2 | 102.1 | 1.2 | 97.2 | 2.3 |
| 60 | 104.2 | 0.7 | 101.7 | 3.0 | 100.6 | 1.0 | 97.2 | 2.2 |

As can be seen from the above dissolution data, the formulas with different specifications all show rapid dissolution in the four media, and can effectively improve the oral absorption of such drugs.

Example 5

The amount of citric acid was 4 mg and 6 mg, and the changes in the pH value of risperidone aqueous solutions with different concentrations are shown in Table 10.

Table 10. Summary of pH values of risperidone aqueous solutions

| Name mg | 4 mg based on anhydrous citric acid | | | 6 mg based on anhydrous citric acid | | |
|---|---|---|---|---|---|---|
| Risperidone | 0.25 | 1 | 2 | 0.25 | 1 | 2 |
| Citric acid monohydrate | 4.375 | 4.375 | 4.375 | 6.6 | 6.6 | 6.6 |

(continued)

| Name mg | 4 mg based on anhydrous citric acid | | | 6 mg based on anhydrous citric acid | | |
|---|---|---|---|---|---|---|
| Purified water | 150 | 150 | 150 | 150 | 150 | 150 |
| pH value | 2.13 | 2.45 | 2.81 | 2.03 | 2.19 | 2.55 |

As can be seen from the above data, in the same citric acid amount system, the pH values of risperidone aqueous solutions with different concentrations fluctuated.

Table 11. Summary table of formulas 13-16

| Name | | Formula 13 | | Formula 14 | | Formula 15 | | Formula 16 | |
|---|---|---|---|---|---|---|---|---|---|
| Specification | | 0.25mg | | 2mg | | 0.25mg | | 2mg | |
| Difference | | 4 mg based on anhydrous citric acid | | | | 6 mg based on anhydrous citric acid | | | |
| Solvent | Purified water* | q.s. | % | q.s. | % | q.s. | % | q.s. | % |
| Flavoring agent | Sucralose | 3 | 6.76 | 3 | 6.76 | 3 | 6.44 | 3 | 6.44 |
| Flavoring agent | Menthol | 2 | 4.51 | 2 | 4.51 | 2 | 4.29 | 2 | 4.29 |
| Flavoring agent | Sodium chloride | 5 | 11.27 | 5 | 11.27 | 5 | 10.73 | 5 | 10.73 |
| Stabilizer | Citric acid monohydrate | 4.375 | 9.86 | 4.375 | 9.86 | 6.6 | 14.16 | 6.6 | 14.16 |
| Plasticizer | Glycerol | 2 | 4.51 | 2 | 4.51 | 2 | 4.29 | 2 | 4.29 |
| Drug substance | Risperidone (API) | 0.25 | 0.56 | 2 | 4.51 | 0.25 | 0.54 | 2 | 4.29 |
| Filler | MCC | 6.75 | 15.21 | 5 | 11.26 | 6.75 | 14.48 | 5 | 10.73 |
| Colorant | Titanium dioxide | 1 | 2.25 | 1 | 2.25 | 1 | 2.15 | 1 | 2.15 |
| Film-forming material | HPMC | 20 | 45.07 | 20 | 45.07 | 20 | 42.92 | 20 | 42.92 |
| Tablet weight mg | | 44.375 | 100 | 44.375 | 100 | 46.6 | 100 | 46.6 | 100 |
| *Purified water was removed after film preparation and drying | | | | | | | | | |

Exemplary preparation method of formulas 13-16:

step 1: a solvent, a flavoring agent, a stabilizer, a plasticizer, a drug substance, a colorant, a filler, and a film-forming material were added according to the formula amount shown in Table 11, electrically stirring or vacuum-emulsifying was performed until uniform, and vacuum deaeration was performed to obtain a drug-containing gel solution;
step 2: the drug-containing gel solution obtained in step 1 was added into a film preparation machine for film coating and drying at a drying parameter of $60\pm10$ °C/20 min to obtain a dry film intermediate;
step 3: the dry film intermediate obtained in step 2 was cut and packaged to obtain the risperidone solid oral film.

The risperidone solid oral film samples of formulas 13-16 were placed at a high temperature of 50 °C for stability testing and taken out on day 10, and the content of related substances was investigated. Summary data are shown in Table 12:

Table 12. Stability summary table of formulas 13-16 at 50 °C

| batch No. | | Formula 13 | | Formula 14 | | Formula 15 | | Formula 16 | |
|---|---|---|---|---|---|---|---|---|---|
| Specification | | 0.25mg | | 2mg | | 0.25mg | | 2mg | |
| Difference | | 4 mg based on anhydrous citric acid | | | | 6 mg based on anhydrous citric acid | | | |
| Lofting conditions | | 0 days | 50 °C, 10 days | 0 days | 50 °C, 10 days | 0 days | 50 °C, 10 days | 0 days | 50 °C, 10 days |
| Name | RRT | % Area | | % Area | | % Area | | % Area | |
| Unknown single impurities | 0.64 | / | / | / | / | / | / | / | / |
| IM-Cis-N-Oxide | 0.65 | / | / | 0.01 | 0.01 | / | / | / | / |
| Unknown single impurities | 0.65 | / | / | / | 0.00 | / | / | / | / |
| Unknown single impurities | 0.84 | / | / | / | / | / | / | / | / |
| Unknown single impurities | 0.98 | / | / | 0.01 | / | / | / | 0.01 | 0.01 |
| API | 1.00 | 100.00 | 100.00 | 99.98 | 99.96 | 99.68 | 99.74 | 99.99 | 99.99 |
| Unknown single impurities | 1.02 | / | / | / | / | 0.32 | 0.26 | / | / |
| Unknown single impurities | 1.06 | / | / | / | / | / | / | / | / |
| Unknown single impurities | 1.10 | / | / | / | 0.00 | / | / | / | / |
| Unknown single impurities | 1.18 | / | / | / | / | / | / | / | / |
| Unknown single impurities | 1.32 | / | / | / | 0.01 | / | / | / | / |
| Total impurity | | 0.00 | 0.00 | 0.02 | 0.02 | 0.32 | 0.26 | 0.01 | 0.01 |

The data show that when the citric acid was 4 mg/tablet, the finished products of both specifications were very stable, and the related substances met the quality criteria of the finished products of the drug substance.

In conclusion, the risperidone solid oral film provided by the present disclosure has good mouthfeel, is easy to accept and use by patients, and has good stability in pharmaceutical part. Moreover, the production cost is low, it is suitable for industrial production, and the environmental pollution is small.

Comparative Example 1

Table 13. Summary table of formula A

| Formula mg | | Formula A | Percentage % |
|---|---|---|---|
| Solvent | Purified water* | q.s. | / |
| Flavoring agent | Sucralose | 2.00 | 4.24 |
| Flavoring agent | Menthol | 1.00 | 2.12 |
| Stabilizer | Anhydrous citric acid | 3.00 | 6.36 |
| Colorant | Titanium dioxide | 0.15 | 0.32 |

**EP 4 778 515 A1**

(continued)

| Formula mg | | Formula A | Percentage % |
|---|---|---|---|
| Plasticizer | Glycerol | 2.00 | 4.24 |
| Drug substance | Risperidone | 3.00 | 6.36 |
| Film-forming material | Hydroxypropyl methylcellulose | 24.00 | 50.9 |
| Filler | Microcrystalline cellulose | 12.00 | 25.45 |
| Tablet weight | | 47.15 | 100 |
| Mouthfeel | | Severe bitter taste, which cannot be masked by a flavoring agent | |

*Purified water was removed after film preparation and drying.

Preparation method of formula A:

step 1: a solvent, a flavoring agent, a stabilizer, a plasticizer, a drug substance, a colorant, and a film-forming material were added according to the formula amount shown in Table 13, electrically stirring or vacuum-emulsifying was performed until uniform, and vacuum deaeration was performed to obtain a drug-containing gel solution;
step 2: the drug-containing gel solution obtained in step 1 was added into a film preparation machine for film coating and drying (at a drying parameter of 60±10 °C/20 min) to obtain a dry film intermediate;
step 3: the dry film intermediate obtained in step 2 was cut and packaged to obtain the risperidone solid oral film.

The obtained product was found after tasting to have a severe bitter taste which could not be masked by a flavoring agent.

Comparative Example 2

Preparation was performed with reference to the method in Example 1 of CN107028917A. The formula is as follows:

Table 14. Summary table of formula B

| Formula mg | Formula B | Percentage % |
|---|---|---|
| Risperidone | 1 | 2.66 |
| Microcrystalline cellulose | 12 | 31.90 |
| Maltodextrin | 4 | 10.63 |
| Sucralose | 1 | 2.66 |
| Citric acid | 0.5 | 1.33 |
| Glycerol | 5 | 13.29 |
| Titanium dioxide | 0.06 | 0.16 |
| Hydroxypropyl methylcellulose E-15 | 14 | 37.21 |
| Menthol | 0.06 | 0.16 |
| Aqueous ethanol solution | q.s. | |
| Mouthfeel | Severe bitter taste, which cannot be masked by a flavoring agent | |

The formula was prepared with reference to the preparation method in CN107028917A. The obtained product was found after tasting to have a severe bitter taste which could not be masked by a flavoring agent.

Comparative Example 3

Preparation was performed with reference to the exemplary method of CN108685876A (Example 3). The formula is as follows:

Table 15. Summary table of formula C

| Formula | Formula C |
|---|---|
| Risperidone, g | 0.8 |
| Purified water, mL | 20 |
| L-Tartaric acid, g | 3 |
| Sucrose, g | 0.2 |
| Sweet orange essence, g | 0.2 |
| Titanium dioxide, g | 0.3 |
| Disodium edetate, g | 0.02 |
| Povidone K30, g | 1.5 |
| Hydroxypropyl methylcellulose E3, g | 2 |
| Hydroxypropyl methylcellulose E15, g | 1 |
| Glycerol, g | 0.098 |
| Mouthfeel | Severe bitter taste, which cannot be masked by a sweetener |

The formula was prepared with reference to the preparation method in CN108685876A. The obtained product was found after tasting to have a severe bitter taste which could not be masked by a flavoring agent.

Moreover, according to the description in paragraph 0006 of the specification of CN113842376A, the formula had poor stability after being stored at a high temperature of 60 °C for 10 days, and unknown single impurities exceeding the limit (actually measured as 0.59% for 5 days, 0.94% for 10 days; limit: 0.2%) would affect the medication safety of patients to a certain extent.

Example 6

The palatability of formulas A, B, and C and formulas 10-16 was evaluated comprehensively by using a bitterness grade evaluation method and a multi-factor investigation evaluation method with reference to the Technical Instruction Principles of Pediatric Drug Palate Design and Evaluation (Draft for Solicitation of Comments) and the literature (Li Pan, Han Xue, Lin Junzhi, Jiang Hong, Yang Ming, Zhang Dingkun, Han Li, Application and Development of Volunteer Sensory Test in Drug Taste Evaluation [J]. Chinese Pharmaceutical Journal, 2017, 52(22):1971-1975).

According to the quality attributes of the oral soluble film, palatability evaluation was performed in the aspects of appearance, bitter taste, sweetness, tingly taste, oral sense of foreign matters, etc., where bitter taste, tingly taste, and oral sense of foreign matters were main evaluation characteristics; appearance and sweetness were bonus characteristics. The grading, score range, and evaluation results are as follows:

Table 16. Mouthfeel evaluation table

| Grade | Description of bitter taste | Description of tingly taste | Description of sense of foreign matters | Score range |
|---|---|---|---|---|
| I | Barely any bitter taste | Barely any tingly sensation in the oral cavity | Barely any sense of foreign matters | 8~10 |
| II | Mild bitter taste | Mild tingly sensation in the oral cavity | Mild sense of foreign matters | 6~8 |

(continued)

| Grade | Description of bitter taste | Description of tingly taste | Description of sense of foreign matters | Score range |
|---|---|---|---|---|
| III | Acceptable bitter taste | Acceptable tingly sensation in the oral cavity | Acceptable sense of foreign matters | 4-6 |
| IV | Tolerable, strong bitterness | Tolerable, strong tingly sensation in the oral cavity | Tolerable, strong sense of foreign matters | 2~4 |
| V | Intolerable bitter taste | Intolerable tingly sensation in the oral cavity | Intolerable sense of foreign matters | 0~2 |

| Grade | Description of appearance | | Description of sweetness | Score range |
|---|---|---|---|---|
| I | Pleasant color and suitable size | | Moderate sweetness | 2~3 |
| II | Good color and acceptable size | | Mildly excessive sweetness or mild tastelessness | 1~2 |
| III | Excessive deep color or excessive size | | Excessive sweetness or tastelessness | 0~1 |

| Score | Description of palatability |
|---|---|
| 30~36 | Pleasant palatability |
| 24~30 | Acceptable palatability |
| 18~24 | Slightly inferior palatability to be improved |
| 12~18 | Relatively inferior and tolerable palatability |
| 0~12 | Very inferior palatability and no flavoring effect |

| Subject | Formula | Bitter taste | Tingly taste | Sense of foreign matters | Appearance | Sweetness | Total score |
|---|---|---|---|---|---|---|---|
| 001 | A | 2 | 4 | 5 | 3 | 0 | 14 |
| | B | 0 | 5 | 5 | 2 | 0 | 12 |
| | C | 0 | 10 | 7 | 3 | 0 | 20 |
| | 10 | 8 | 10 | 9 | 3 | 3 | 33 |
| | 11 | 8 | 10 | 7 | 3 | 3 | 31 |
| | 12 | 9 | 10 | 5 | 3 | 3 | 30 |
| | 13 | 10 | 10 | 7 | 3 | 2 | 32 |
| | 14 | 8 | 10 | 7 | 3 | 2 | 30 |
| | 15 | 9 | 10 | 7 | 3 | 2 | 31 |
| | 16 | 9 | 10 | 7 | 3 | 2 | 31 |

(continued)

| Subject | Formula | Bitter taste | Tingly taste | Sense of foreign matters | Appearance | Sweetness | Total score |
|---------|---------|--------------|--------------|--------------------------|------------|-----------|-------------|
| 002 | A | 1 | 2 | 4 | 2 | 0 | 9 |
| | B | 2 | 3 | 4 | 2 | 0 | 11 |
| | C | 0 | 10 | 5 | 3 | 0 | 18 |
| | 10 | 9 | 10 | 8 | 3 | 3 | 33 |
| | 11 | 8 | 10 | 7 | 3 | 3 | 31 |
| | 12 | 9 | 10 | 5 | 3 | 3 | 30 |
| | 13 | 9 | 10 | 8 | 3 | 3 | 33 |
| | 14 | 8 | 10 | 8 | 3 | 3 | 32 |
| | 15 | 8 | 10 | 8 | 3 | 3 | 32 |
| | 16 | 9 | 10 | 8 | 3 | 3 | 33 |
| 003 | A | 1 | 3 | 6 | 3 | 0 | 13 |
| | B | 1 | 4 | 6 | 3 | 0 | 14 |
| | C | 0 | 10 | 6 | 3 | 0 | 19 |
| | 10 | 8 | 10 | 10 | 3 | 3 | 34 |
| | 11 | 8 | 10 | 5 | 3 | 3 | 29 |
| | 12 | 9 | 10 | 5 | 3 | 3 | 30 |
| | 13 | 8 | 10 | 9 | 3 | 2 | 32 |
| | 14 | 9 | 10 | 9 | 3 | 2 | 33 |
| | 15 | 8 | 10 | 9 | 3 | 2 | 32 |
| | 16 | 9 | 10 | 9 | 3 | 2 | 33 |
| 004 | A | 1 | 5 | 6 | 3 | 1 | 16 |
| | B | 1 | 5 | 5 | 3 | 1 | 15 |
| | C | 1 | 10 | 6 | 3 | 1 | 21 |
| | 10 | 9 | 10 | 8 | 3 | 3 | 33 |
| | 11 | 9 | 10 | 7 | 3 | 3 | 32 |
| | 12 | 8 | 10 | 4 | 3 | 3 | 28 |
| | 13 | 9 | 10 | 8 | 3 | 3 | 33 |
| | 14 | 9 | 10 | 8 | 3 | 3 | 33 |
| | 15 | 9 | 10 | 8 | 3 | 3 | 33 |
| | 16 | 9 | 10 | 8 | 3 | 3 | 33 |

(continued)

| Subject | Formula | Bitter taste | Tingly taste | Sense of foreign matters | Appearance | Sweetness | Total score |
|---|---|---|---|---|---|---|---|
| 005 | A | 2 | 2 | 5 | 3 | 0 | 12 |
| | B | 1 | 2 | 6 | 3 | 0 | 12 |
| | C | 1 | 10 | 6 | 3 | 0 | 20 |
| | 10 | 9 | 10 | 10 | 3 | 3 | 35 |
| | 11 | 8 | 10 | 8 | 3 | 3 | 32 |
| | 12 | 8 | 10 | 6 | 3 | 3 | 30 |
| | 13 | 9 | 10 | 7 | 3 | 3 | 32 |
| | 14 | 9 | 10 | 7 | 3 | 3 | 32 |
| | 15 | 8 | 10 | 7 | 3 | 2 | 30 |
| | 16 | 10 | 10 | 7 | 3 | 2 | 32 |

The above formulas were evaluated for mouthfeel, and the bitter taste was not removed in all of the formulas compared with formulas A, B and C, and the mouthfeel was to be improved. However, formulas 10-16 of the present disclosure had acceptable mouthfeel, no bitter taste, and were very outstanding.

Example 7. Stability Test of Formulas 11 and 12

The samples with inner packaging (polyester/aluminum/cast polyethylene pharmaceutical composite film) were separately placed under high temperature (50 °C) and high humidity (90% RH$\pm$5% RH) conditions, and sampled for detection on days 0, 5, 10, and 30. The samples without packaging were placed under a high temperature (50 °C), and sampled for detection on days 0, 5, 10, and 30. Also, the samples without packaging boxes and with inner packaging were placed under a light condition (total illumination $\geq 1.2 \times 10^6$ lux·hr, total ultraviolet amplitude $\geq 200$ W·hr/m$^2$), and sampled for detection on day 10. The samples with inner packaging (polyester/aluminum/cast polyethylene pharmaceutical composite film) were sampled for detection under an acceleration condition (40 °C$\pm$2 °C, 75% RH$\pm$5% RH) in months 0, 1, 3, and 6. The samples with inner packaging (polyester/aluminum/cast polyethylene pharmaceutical composite film) were sampled for detection under a long-term condition (25 °C$\pm$2 °C, 60% RH$\pm$5% RH) in months 0, 3, and 6.

1. Influencing factor stability test

EP 4 778 515 A1

Table 17. Influencing factor stability data summary of formula 11 (1 mg)

| batch No. | | | | Formula 11 (1 mg specification) | | | | | | | | | | | |
|---|---|---|---|---|---|---|---|---|---|---|---|---|---|---|---|
| Test item | | Limit requirement | 0 days | High temperature (50 °C) | | | High temperature (50 °C) | | | High humidity (90% RH±5% RH) | | Illumination (total illumination ≥ $1.2 \times 10^6$ lux·hr, total ultraviolet amplitude ≥ 200 W·hr/m$^2$) | |
| | | | | Without packaging | | | With packaging | | | | | | Without packaging |
| | | | | 5 days | 10 days | 30 days | 5 days | 10 days | 30 days | 5 days | 10 days | 10 days | 10 days |
| Appearance | Appearance | This product is a white or off-white flaky membrane. | This product is an off-white flaky membrane. | This product is an off-white flaky membrane. | This product is an off-white flaky membrane. | This product is an off-white flaky membrane. | This product is an off-white flaky membrane. | This product is an off-white flaky membrane. | This product is an off-white flaky membrane. | This product is an off-white flaky membrane. | This product is an off-white flaky membrane. | This product is an off-white flaky membrane. | This product is an off-white flaky membrane. |
| Identification | -1 | In the spectrum recorded under the content determination item, the ultraviolet absorption spectrum of the main peak of the test sample solution should be consistent with that of the control solution. | Comply with the standard | / | / | / | / | / | / | / | / | / | / |

EP 4 778 515 A1

(continued)

| batch No. | | Formula 11 (1 mg specification) | | | | | | | | | | | |
|---|---|---|---|---|---|---|---|---|---|---|---|---|---|
| Test item | | Limit requirement | 0 days | High temperature (50 °C) | | | High temperature (50 °C) | | | High humidity (90% RH±5% RH) | | Illumination (total illumination ≥ 1.2 × 10⁶ lux·hr, total ultraviolet amplitude ≥ 200 W·hr/m²) | |
| | | | | Without packaging | | | With packaging | | | | | | Without packaging |
| | | | | 5 days | 10 days | 30 days | 5 days | 10 days | 30 days | 5 days | 10 days | 10 days | 10 days |
| | -2 | In the chromato-gram recorded under the con-tent determina-tion item, the re-tention time of the main peak of the test sample solution should be consistent with that of the main peak of the control solution. | Comply with the standard | / | / | / | / | / | / | / | / | / | / |
| Inspection | Related sub-stances | Bicyclorisperi-don e (IM-Bicy-clo) should not be more than 0.3% | Undetect-able | Undetect-able | Undetect-able | Undetect-able | Undetectable | Undetect-able | Undetect-able | Undetectable | Undetect-able | Undetect-able | Undetect-able |

| Test item | | Limit requirement | 0 days | High temperature (50 °C) | | | High temperature (50 °C) | | | High humidity (90% RH±5% RH) | | Illumination (total illumination ≥ 1.2 × 10⁶ lux·hr, total ultraviolet amplitude ≥ 200 W·hr/m²) | |
|---|---|---|---|---|---|---|---|---|---|---|---|---|---|
| batch No. | | | Formula 11 (1 mg specification) | | | | | | | | | | |
| | | | | Without packaging | | | With packaging | | | | | | Without packaging |
| | | | | 5 days | 10 days | 30 days | 5 days | 10 days | 30 days | 5 days | 10 days | 10 days | 10 days |
| | | Risperidone cis-nitrogen oxide (IM-Cis-N-Oxide) should not be more than 0.5% | 0.01% | 0.01% | Undetectable | 0.01% | 0.01 | 0.01% | 0.02% | 0.01% | 0.01% | 0.01% | 0.15% |
| | | Other single impurities should not be more than 0.2% | 0.03%(RRT =0.96) | Undetectable (0.01%) (RRT = 0.98) | Undetectable (0.01%) (RRT = 0.98) | Undetectable (0.02%) (RRT = 0.98) | Undetectable (0.01%) (RRT = 0.81/0.96/0.98) | Undetectable (0.01%) (RRT = 0.81/0.98) | Undetectable (0.01%) (RRT = 0.98) | Undetectable (0.01%) (RRT = 0.81/0.96/0.98) | Undetectable (0.01%) (RRT = 0.98) | Undetectable (0.01%) (RRT = 0.98) | 1.60% (RRT=1.04) |
| | | Total impurities should not be more than 1.0% | 0.04% | 0.01% | 0.00% | 0.01% | 0.01% | 0.01% | 0.02% | 0.01% | 0.01% | 0.01% | 3.96% |
| | | Limit of disregarding is 0.03% | / | / | / | / | / | / | / | / | / | / | / |
| | Content homogenicity | Should comply with the standard (A+2.2S≤15.0) | Comply with the standard (A + 2.2S = 5.6) | / | / | / | / | / | / | / | / | / | / |
| | Dissolution rate | The dissolution amount of 30 minutes should be 80% of the labeled amount | 99% | 100% | 97% | 99% | 101% | 96% | 100% | 100% | 97% | 98% | / |
| | Water content | Report results | 4.60% | 4.00% | 4.20% | 3.40% | 4.00% | 4.20% | 4.60% | 3.90% | 4.20% | 4.20% | / |

(continued)

| batch No. | Formula 11 (1 mg specification) | | | | | | | | | | | |
|---|---|---|---|---|---|---|---|---|---|---|---|---|
| Test item | Limit requirement | 0 days | High temperature (50 °C) | | | High temperature (50 °C) | | | High humidity (90% RH±5% RH) | | Illumination (total illumination ≥ 1.2 × $10^6$ lux·hr, total ultraviolet amplitude ≥ 200 W·hr/$m^2$) | |
| | | | Without packaging | | | With packaging | | | | | | Without packaging |
| | | | 5 days | 10 days | 30 days | 5 days | 10 days | 30 days | 5 days | 10 days | 10 days | 10 days |
| Content determination | This product should contain 90.0%-110.0% of the labeled amount of risperidone ($C_{23}H_{27}FN_4O_2$). | 100.30% | 99.30% | 99.20% | 99.40% | 99.60% | 99.10% | 98.80% | 99.20% | 99.20% | 99.00% | / |

24

Formula 11 with inner packaging or without inner packaging was stable at a high temperature of 50 °C; formula 11 with inner packaging was stable under high humidity and light conditions.

Table 18. Influencing factor stability data summary of formula 12 (2 mg)

| batch No. | | | | Formula 12 (2 mg specification) | | | | | | | | | | |
|---|---|---|---|---|---|---|---|---|---|---|---|---|---|---|
| Test item | | Limit requirement | 0 days | High temperature (50 °C) | | | High temperature (50 °C) | | | High humidity (90% RH ±5% RH) | | Illumination (total illumination ≥ $1.2 \times 10^6$ lux·hr, total ultraviolet amplitude ≥ 200 W·hr/m$^2$) | |
| | | | | Without packaging | | | With packaging | | | | | | Without packaging |
| | | | | 5 days | 10 days | 30 days | 5 days | 10 days | 30 days | 5 days | 10 days | 10 days | 10 days |
| Appearance | Appearance | This product is a white or off-white flaky membrane. | This product is an off-white flaky membrane. | This product is an off-white flaky membrane. | This product is an off-white flaky membrane. | This product is an off-white flaky membrane. | This product is an off-white flaky membrane. | This product is an off-white flaky membrane. | This product is an off-white flaky membrane. | This product is an off-white flaky membrane. | This product is an off-white flaky membrane. | This product is an off-white flaky membrane. | This product is an off-white flaky membrane. |
| Identification | -1 | In the spectrum recorded under the content determination item, the ultraviolet absorption spectrum of the main peak of the test sample solution should be consistent with that of the control solution. | Comply with the standard | / | / | / | / | / | / | / | / | / | / |
| | -2 | In the chromatogram recorded under the content determination item, the retention time of the main peak of the test sample solution should be consistent with that of the main peak of the control solution. | Comply with the standard | / | / | / | / | / | / | / | / | / | / |

| batch No. | | | | Formula 12 (2 mg specification) | | | | | | | | | | |
|---|---|---|---|---|---|---|---|---|---|---|---|---|---|---|
| Test item | | Limit requirement | 0 days | High temperature (50 °C) | | | High temperature (50 °C) | | | High humidity (90% RH ±5% RH) | | Illumination (total illumination ≥ 1.2 × 10⁶ lux·hr, total ultraviolet amplitude ≥ 200 W·hr/m²) | |
| | | | | Without packaging | | | With packaging | | | | | | Without packaging |
| | | | | 5 days | 10 days | 30 days | 5 days | 10 days | 30 days | 5 days | 10 days | 10 days | 10 days |
| Appearance | Appearance | This product is a white or off-white flaky membrane. | This product is an off-white flaky membrane. | This product is an off-white flaky membrane. | This product is an off-white flaky membrane. | This product is an off-white flaky membrane. | This product is an off-white flaky membrane. | This product is an off-white flaky membrane. | This product is an off-white flaky membrane. | This product is an off-white flaky membrane. | This product is an off-white flaky membrane. | This product is an off-white flaky membrane. | This product is an off-white flaky membrane. |
| Inspection | Related substances | Bicyclorisperidon e (IM-Bicyclo) should not be more than 0.3% | Undetectable | Undetectable | Undetectable | Undetectable | Undetectable | Undetectable | Undetectable | Undetectable | Undetectable | Undetectable | Undetectable |
| | | Risperidone cis-nitrogen oxide (IM-Cis-N-Oxide) should not be more than 0.5% | 0.01% | 0.01% | 0.01% | 0.01% | 0.01% | 0.03% | 0.03% | 0.01% | 0.01% | 0.01% | 0.14% |
| | | Other single impurities should not be more than 0.2% | 0.03% (RRT=0.96) | Undetectable (0.01%) (RRT = 0.96/0.98) | Undetectable (0.01%) (RRT = 0.81/0.84/0.98) | Undetectable (0.02%) (RRT = 0.82) | Undetectable (0.01%) (RRT = 0.96/0.98) | Undetectable (0.01%) (RRT = 0.98) | Undetectable (0.01%) (RRT = 0.98) | Undetectable (0.02%) (RRT = 0.96) | Undetectable (0.01%) (RRT = 0.81) | Undetectable (0.01%) (RRT = 0.81/0.98) | 0.97% (RRT=1.04) |
| | | Total impurities should not be more than 1.0% | 0.04% | 0.01% | 0.01% | 0.01% | 0.01 | 0.03% | 0.03% | 0.01% | 0.01% | 0.01% | 2.84% |
| | | Limit of disregarding is 0.03% | / | / | / | / | / | / | / | / | / | / | / |
| | Content homogenicity | Should comply with the standard (A+2.2S≤ 15.0) | Comply with the standard (A + 2.2S = 4.5) | / | / | / | / | / | / | / | / | / | / |

EP 4 778 515 A1

(continued)

| batch No. | | Formula 12 (2 mg specification) | | | | | | | | | | | | |
|---|---|---|---|---|---|---|---|---|---|---|---|---|---|---|
| Test item | | Limit requirement | 0 days | High temperature (50 °C) | | | High temperature (50 °C) | | | High humidity (90% RH ±5% RH) | | Illumination (total illumination ≥ 1.2 × 10$^6$ lux·hr, total ultraviolet amplitude ≥ 200 W·hr/m$^2$) | |
| | | | | Without packaging | | | With packaging | | | | | | Without packaging |
| | | | | 5 days | 10 days | 30 days | 5 days | 10 days | 30 days | 5 days | 10 days | 10 days | 10 days |
| Appearance | Appearance | This product is a white or off-white flaky membrane. | This product is an off-white flaky membrane. | This product is an off-white flaky membrane. | This product is an off-white flaky membrane. | This product is an off-white flaky membrane. | This product is an off-white flaky membrane. | This product is an off-white flaky membrane. | This product is an off-white flaky membrane. | This product is an off-white flaky membrane. | This product is an off-white flaky membrane. | This product is an off-white flaky membrane. | This product is an off-white flaky membrane. |
| | Dissolution rate | The dissolution amount of 30 minutes should be 80% of the labeled amount | 98% | 98% | 96% | 98% | 98% | 94% | 97% | 98% | 95% | 96% | / |
| | Water content | Report results | 5.80% | 3.40% | 3.40% | 2.60% | 5.00% | 5.30% | 5.10% | 5.30% | 5.20% | 5.30% | / |
| Content determination | | This product should contain 90.0%-110.0% of the labeled amount of risperidone ($C_{23}H_{27}FN_4O_2$). | 98.60% | 97.60% | 96.20% | 97.20% | 96.00% | 96.90% | 97.60% | 96.80% | 97.30% | 96.30% | / |

Formula 12 with inner packaging or without inner packaging was stable at a high temperature of 50 °C; formula 12 with inner packaging was stable under high humidity and light conditions.

2. Accelerated stability test

Table 19. Accelerated stability data summary of formula 11 (1 mg)

| batch No. | | Formula 11 (1 mg specification) | | | | |
|---|---|---|---|---|---|---|
| Test item | | Limit requirement | 0 months | 1 months | 3 months | 6 months |
| Appearance | Appearance | This product is a white or off-white flaky membrane. | This product is an off-white flaky membrane. | This product is an off-white flaky membrane. | This product is an off-white flaky membrane. | This product is an off-white flaky membrane. |
| Identification | -1 | In the spectrum recorded under the content determination item, the ultraviolet absorption spectrum of the main peak of the test sample solution should be consistent with that of the control solution. | Comply with the standard | / | / | / |
| | -2 | In the chromatogram recorded under the content determination item, the retention time of the main peak of the test sample solution should be consistent with that of the main peak of the control solution. | Comply with the standard | / | / | / |
| Inspection | Related substances | Bicyclorisperidone (IM-Bicyclo) should not be more than 0.3% | Undetectable | Undetectable | Undetectable | Undetectable |
| | | Risperidone cis-nitrogen oxide (IM-Cis-N-Oxide) should not be more than 0.5% | 0.01% | 0.01% | 0.04% | 0.04% |
| | | Other single impurities should not be more than 0.2% | 0.03%(RRT=0.96) | 0.01% (<limit of disregarding) (RRT = 0.98) | 0.03%(RRT=1.14) | 0.17%(RRT=1.14) |
| | | Total impurities should not be more than 1.0% | 0.04% | 0.01% | 0.07% | 0.21% |
| | Content homogenicity | Should comply with the standard | Comply with the standard (A + 2.2S = 5.6) | / | / | / |
| | | (A+2.2S≤15.0) | | | | |
| | Dissolution rate | The dissolution amount of 30 minutes should be 80% of the labeled amount | 99% | 97% | 99% | 103% |
| | Water content | Report results | 4.60% | 4.40% | 4.10% | 4.30% |

EP 4 778 515 A1

| batch No. | | Formula 11 (1 mg specification) | | | | |
|---|---|---|---|---|---|---|
| Test item | | Limit requirement | 0 months | 1 months | 3 months | 6 months |
| Appearance | Appearance | This product is a white or off-white flaky membrane. | This product is an off-white flaky membrane. | This product is an off-white flaky membrane. | This product is an off-white flaky membrane. | This product is an off-white flaky membrane. |
| Content deter-mination | This product should contain 90.0%-110.0% of the labeled amount of risperidone ($C_{23}H_{27}FN_4O_2$). | 100.30% | 99.40% | 99.60% | 98.90% | |
| Note: the limit of disregarding under the item of related substances is 0.03%. | | | | | | |

Formula 11 (1 mg specification) was placed under the proposed packaging conditions (polyester/aluminum/cast polyethylene pharmaceutical composite film (inner packaging)) at 40 °C±2 °C and 75% RH±5% RH for 6 months, and compared with those in 0 months, there were no significant differences in the investigation indexes, which were all within the acceptable criteria.

.Table 20. Accelerated stability data summary of formula 12 (2 mg)

| batch No. | | Formula 12 (2 mg specification) | | | | |
|---|---|---|---|---|---|---|
| Test item | | Limit requirement | 0 months | 1 months | 3 months | 6 months |
| Appearance | Appearance | This product is a white or off-white flaky membrane. | This product is an off-white flaky membrane. | This product is an off-white flaky membrane. | This product is an off-white flaky membrane. | This product is an off-white flaky membrane. |
| Identification | -1 | In the spectrum recorded under the content determination item, the ultraviolet absorption spectrum of the main peak of the test sample solution should be consistent with that of the control solution. | Comply with the standard | / | / | / |
| | -2 | In the chromatogram recorded under the content determination item, the retention time of the main peak of the test sample solution should be consistent with that of the main peak of the control solution. | Comply with the standard | / | / | / |
| Inspection | Related substances | Bicyclorisperidone (IM-Bicyclo) should not be more than 0.3% | Undetectable | Undetectable | Undetectable | Undetectable |
| | | Risperidone cis-nitrogen oxide (IM-Cis-N-Oxide) should not be more than 0.5% | 0.01% | 0.01% | 0.07% | 0.05% |
| | | Other single impurities should not be more than 0.2% | 0.03%(RRT=0.96) | 0.01% (<limit of disregarding) (RRT = 0.81/0.98/1.31) | 0.05% (RRT=1.14) | 0.21% (RRT=1.14) |
| | | Total impurities should not be more than 1.0% | 0.04% | 0.01% | 0.12% | 0.31% |
| | Content homogenicity | Should comply with the standard | Comply with the standard (A + 2.2S = 4.5) | / | / | / |
| | | (A+2.2S≤15.0) | | | | |
| | Dissolution rate | The dissolution amount of 30 minutes should be 80% of the labeled amount | 98% | 97% | 96% | 96% |
| | Water content | Report results | 5.80% | 5.30% | 5.50% | 5.00% |
| Content determination | | This product should contain 90.0%-110.0% of the labeled amount of risperidone ($C_{23}H_{27}FN_4O_2$). | 98.60% | 98.70% | 95.00% | 97.10% |

Note: the limit of disregarding under the item of related substances is 0.03%

Formula 12 (2 mg specification) was placed under the proposed packaging conditions (polyester/aluminum/cast polyethylene pharmaceutical composite film (inner packaging)) at 40 °C±2 °C and 75% RH±5% RH for 6 months, and compared with those in 0 months, there were no significant differences in the investigation indexes, which were all within the acceptable criteria.

3. Long-term stability test

Table 21. Long-term condition stability data summary of formula 11 (1 mg)

| batch No. | Formula 11 (1 mg specification) | | | |
|---|---|---|---|---|
| **Test item** | **Limit requirement** | **0 months** | **3 months** | **6 months** |
| Appearance | Appearance | This product is a white or off-white flaky membrane. | This product is an off-white flaky membrane. | This product is an off-white flaky membrane. | This product is an off-white flaky membrane. |
| Identification | -1 | In the spectrum recorded under the content determination item, the ultraviolet absorption spectrum of the main peak of the test sample solution should be consistent with that of the control solution. | Comply with the standard | / | / |
| | -2 | In the chromatogram recorded under the content determination item, the retention time of the main peak of the test sample solution should be consistent with that of the main peak of the control solution. | Comply with the standard | / | / |
| Inspection | Related substances | Bicyclorisperidone (IM-Bicyclo) should not be more than 0.3% | Undetectable | Undetectable | Undetectable |
| | | Risperidone cis-nitrogen oxide (IM-Cis-N-Oxide) should not be more than 0.5% | 0.01% | 0.04% | Undetectable |

(continued)

| Appearance | Appearance | This product is a white or off-white flaky membrane. | This product is an off-white flaky membrane. | This product is an off-white flaky membrane. | This product is an off-white flaky membrane. |
|---|---|---|---|---|---|
| | | Other single impurities should not be more than 0.2% | 0.03%(RRT=0.96) | 0.01 (<limit of disregarding) (RRT = 0.81; 0.98) | Undetectable |
| | | Total impurities should not be more than 1.0% | 0.04% | 0.04% | Undetectable |
| | Content homogenicity | Should comply with the standard | Comply with the standard (A + 2.2S = 5.6) | / | / |
| | | (A+2.2S≤15.0) | | | |
| | Dissolution rate | The dissolution amount of 30 minutes should be 80% of the labeled amount | 99% | 99% | 100% |
| | Water content | Report results | 4.60% | 3.70% | 4.20% |
| Content determination | | This product should contain 90.0%-110.0% of the labeled amount of risperidone ($C_{23}H_{27}FN_4O_2$). | 100.30% | 99.40% | 98.50% |
| Note: the limit of disregarding under the item of related substances is 0.03% | | | | | |

Formula 11 (1 mg specification) was placed under the proposed packaging conditions (polyester/aluminum/cast polyethylene pharmaceutical composite film (inner packaging)) at 25 °C±2 °C and 60% RH±5% RH for 6 months, and compared with those in 0 months, there were no significant differences in the investigation indexes, which were all within the acceptable criteria.

Table 22. Long-term condition stability data summary of formula 12 (2 mg)

| batch No. | | Formula 12 (2 mg specification) | | | |
|---|---|---|---|---|---|
| Test item | | Limit requirement | 0 months | 3 months | 6 months |
| Appearance | Appearance | This product is a white or off-white flaky membrane. | This product is an off-white flaky membrane. | This product is an off-white flaky membrane. | This product is an off-white flaky membrane. |

(continued)

| batch No. | | Formula 12 (2 mg specification) | | | |
|---|---|---|---|---|---|
| Test item | | Limit requirement | 0 months | 3 months | 6 months |
| Identification | -1 | In the spectrum recorded under the content determination item, the ultraviolet absorption spectrum of the main peak of the test sample solution should be consistent with that of the control solution. | Comply with the standard | / | / |
| | -2 | In the chromatogram recorded under the content determination item, the retention time of the main peak of the test sample solution should be consistent with that of the main peak of the control solution. | Comply with the standard | / | / |
| Inspection | Related substances | Bicyclorisperidone (IM-Bicyclo) should not be more than 0.3% | Undetectable | Undetectable | Undetectable |
| | | Risperidone cis-nitrogen oxide (IM-Cis-N-Oxide) should not be more than 0.5% | 0.01% | 0.03% | Undetectable |
| | | Other single impurities should not be more than 0.2% | 0.03%(RRT=0.96) | 0.01 (<limit of disregarding) (RRT = 0.81; 0.98) | Undetectable |
| | | Total impurities should not be more than 1.0% | 0.04% | 0.03% | Undetectable |
| | Content homogenicity | Should comply with the standard | Comply with the standard (A + 2.2S = 4.5) | / | / |
| | | (A+2.2S≤15.0) | | | |
| | Dissolution rate | The dissolution amount of 30 minutes should be 80% of the labeled amount | 98% | 95% | 98% |
| | Water content | Report results | 5.80% | 4.80% | 5.20% |
| Content determination | | This product should contain 90.0%-110.0% of the labeled amount of risperidone ($C_{23}H_{27}FN_4O_2$). | 98.60% | 96.50% | 97.50% |
| Note: the limit of disregarding under the item of related substances is 0.03% | | | | | |

Formula 12 (2 mg specification) was placed under the proposed packaging conditions (polyester/aluminum/cast polyethylene pharmaceutical composite film (inner packaging)) at 25 °C±2 °C and 60% RH±5% RH for 6 months, and compared with those in 0 months, there were no significant differences in the investigation indexes, which were all within the acceptable criteria.

Example 8. Tests of mechanical strength and disintegration time limit of formulas 11 and 12

Tensile strength test:

Mechanical performance: a tensile test module was installed after startup, force calibration and height calibration were performed, and the height was set to 2 mm.

The thickness and width of the oral soluble films were measured using a thickness gauge and a ruler, and the cross-sectional areas ($mm^2$) of the oral soluble films were calculated. The measurement was performed in 6 replicates.

The oral soluble films after the thickness and width were measured were fixed on a texture analyzer such that the measured cross section was perpendicular to the stretching direction of the instrument, and the maximum force and the height of the sample were recorded.

$$\text{Tensile strength (MPa)} = \text{maximum force (gf)} \div 102 \div \text{cross-sectional area (mm}^2\text{)}.$$

$$\text{Percent elongation} = \text{maximum force distance (mm)} \div \text{sample height (mm)} \times 100\%.$$

Small-amount solvent disintegration time limit test: the sample was placed in a culture dish containing 20 mL of phosphate buffer solution with pH of 6.8 at a temperature of $(37\pm0.5)$ °C, and the time when the sample was completely dissolved was recorded as the disintegration time. The packaging in Tables 23-24 refers to inner packaging, polyester/-aluminum/cast polyethylene pharmaceutical composite films.

Table 23. Mechanical strength and small-amount solvent disintegration summary of formula 11 during influencing factors

| batch No. | Formula 11 (1 mg specification) | | |
|---|---|---|---|
| Item | Tensile strength/Mpa | Percent elongation | Disintegration time limit (min) |
| 0 days | 12.9 | 9% | 6.69 |
| High temperature 50 °C-30 days-with packaging | 11.0 | 8% | 10.02 |
| High temperature 50 °C-30 days-without packaging | 14.5 | 9% | 5.32 |
| High temperature 60 °C-30 days-with packaging | 10.7 | 8% | 9.88 |
| High temperature 60 °C-30 days-without packaging | 14.5 | 8% | 5.33 |
| High humidity 10 days-with packaging | 12.9 | 9% | 5.58 |
| Illumination 10 days-with packaging | 13.1 | 8% | 5.89 |
| Illumination 10 days-without packaging | 14.0 | 7% | 5.42 |

Table 24. Mechanical strength and small-amount solvent disintegration summary of formula 12 during influencing factors

| batch No. | Formula 12 (2 mg specification) | | |
|---|---|---|---|
| Item | Tensile strength/Mpa | Percent elongation | Disintegration time limit (min) |
| 0 days | 11.4 | 8% | 4.38 |
| High temperature 50 °C-30 days-with packaging | 12.1 | 11% | 5.51 |
| High temperature 50 °C-30 days-without packaging | 16.1 | 11% | 5.48 |
| High temperature 60 °C-30 days-with packaging | 12.4 | 11% | 5.09 |
| High temperature 60 °C-30 days-without packaging | 16.4 | 12% | 5.44 |
| High humidity 10 days-with packaging | 12.7 | 10% | 5.05 |

(continued)

| batch No. | Formula 12 (2 mg specification) | | |
|---|---|---|---|
| Item | Tensile strength/Mpa | Percent elongation | Disintegration time limit (min) |
| Illumination 10 days-with packaging | 12.8 | 9% | 4.41 |
| Illumination 10 days-without packaging | 15.7 | 9% | 4.32 |

The embodiments of the present disclosure have been described above. However, the embodiments described above are not intended to limit the present disclosure. Any modification, equivalent replacement, improvement, and the like made without departing from the spirit and principle of the present disclosure shall fall within the protection scope of the present disclosure.

**Claims**

1.  A risperidone solid oral film, wherein the risperidone solid oral film in each dosage unit comprises: 0.10%-8.00% of risperidone, 5.00%-40.00% of a flavoring agent, 5.00%-40.00% of a stabilizer, 2.00%-15.00% of a plasticizer, 0-45.00% of a filler, 2.00%-8.00% of a colorant, and 25.00%-70.00% of a film-forming material; the percentage refers to the percentage of the mass of each of the components relative to the total mass of the risperidone solid oral film; the risperidone solid oral film does not comprise polyvinylpyrrolidone and a coating material.

2.  The risperidone solid oral film as claimed in claim 1, wherein the risperidone solid oral film in each dosage unit comprises 0.1 mg-3 mg of risperidone, such as 0.25 mg, 1 mg, or 2 mg of risperidone.

3.  The risperidone solid oral film as claimed in claim 1, wherein:

    the flavoring agent is selected from one or more of sucrose, mannitol, dextran, sucralose, aspartame, menthol, and sodium chloride;
    and/or,
    the stabilizer is selected from one or more of citric acid and/or sodium citrate, tartaric acid, hydrochloric acid, and malic acid;
    and/or,
    the plasticizer is selected from one or more of glycerol, propylene glycol, sorbitol, and polyethylene glycol;
    and/or,
    the filler is selected from one or more of microcrystalline cellulose, mannitol, starch, pregelatinized starch, maltodextrin, sucrose, lactose, sorbitol, and glucose;
    and/or,
    the colorant is selected from titanium dioxide;
    and/or,
    the film-forming material is selected from hydroxypropyl methylcellulose and/or polyvinyl alcohol.

4.  The risperidone solid oral film as claimed in claim 3, wherein:

    the content of the flavoring agent is 5.00%-40.00%, preferably 6.00%-35.00%; the percentage refers to the percentage of the mass of the flavoring agent relative to the total mass of the risperidone solid oral film;
    and/or,
    the content of the stabilizer is 5.00%-40.00%, preferably 5.00%-35.00%; the percentage refers to the percentage of the mass of the stabilizer relative to the total mass of the risperidone solid oral film;
    and/or,
    the content of the plasticizer is 2.00%-15.00%, preferably 2.00%-13.00%; the percentage refers to the percentage of the mass of the plasticizer relative to the total mass of the risperidone solid oral film;
    and/or,
    the content of the filler is 0-45.00%, preferably 0-43.00%; the percentage refers to the percentage of the mass of the filler relative to the total mass of the risperidone solid oral film;
    and/or,
    the content of the colorant is 2.00%-8.00%, preferably 2.00%-7.00%; the percentage refers to the percentage of the mass of the colorant relative to the total mass of the risperidone solid oral film;
and/or,

the content of the film-forming material is 25.00%-70.00%, preferably 25.00%-65.00%; the percentage refers to the percentage of the mass of the film-forming material relative to the total mass of the risperidone solid oral film; preferably, for the risperidone solid oral film, the oral film in each dosage unit comprises or consists essentially of the following components:

1.00%-3.00% of risperidone, 5.00%-10.00% of a flavoring agent, 5.00%-10.00% of a stabilizer, 7.00%-13.00% of a plasticizer, 35.00%-45.00% of a filler, 2.00%-5.00% of a colorant, and 25.00%-40.00% of a film-forming material; the percentage refers to the percentage of the mass of each of the components relative to the total mass of the risperidone solid oral film; the risperidone solid oral film does not comprise polyvinylpyrrolidone and a coating material; preferably, the flavoring agent consists of sucralose and sodium chloride, the stabilizer is citric acid, the plasticizer is glycerol, the filler is microcrystalline cellulose, the colorant is titanium dioxide, and the film-forming material is hydroxypropyl methylcellulose.

**5.** The risperidone solid oral film as claimed in claim 1, wherein:
the risperidone solid oral film is selected from any one of the following formulas:

formula 1: 5.56% risperidone, 8.33% sucralose, 2.77% menthol, 13.89% sodium chloride, 5.56% anhydrous citric acid, 5.56% glycerol, 2.77% titanium dioxide, and 55.56% hydroxypropyl methylcellulose, wherein the percentage refers to the percentage of the mass of each of the components relative to the total mass of the risperidone solid oral film;

formula 2: 5.41% risperidone, 8.11% sucralose, 13.51% sodium chloride, 10.81% anhydrous citric acid, 5.41% glycerol, 2.70% titanium dioxide, and 54.05% hydroxypropyl methylcellulose, wherein the percentage refers to the percentage of the mass of each of the components relative to the total mass of the risperidone solid oral film;

formula 3: 5.41% risperidone, 8.10% sucralose, 5.41% menthol, 13.51% sodium chloride, 5.41% anhydrous citric acid, 2.70% glycerol, 5.41% titanium dioxide, and 54.05% hydroxypropyl methylcellulose, wherein the percentage refers to the percentage of the mass of each of the components relative to the total mass of the risperidone solid oral film;

formula 4: 5.41% risperidone, 8.10% sucralose, 5.41% menthol, 13.51% sodium chloride, 5.41% anhydrous citric acid, 2.70% glycerol, 5.41% titanium dioxide, and 54.05% polyvinyl alcohol, wherein the percentage refers to the percentage of the mass of each of the components relative to the total mass of the risperidone solid oral film;

formula 5: 5.41% risperidone, 8.10% sucralose, 5.41% menthol, 13.51% sodium chloride, 5.41% anhydrous citric acid, 5.41% glycerol, 2.70% titanium dioxide, and 54.05% hydroxypropyl methylcellulose, wherein the percentage refers to the percentage of the mass of each of the components relative to the total mass of the risperidone solid oral film;

formula 6: 5.13% risperidone, 7.69% sucralose, 5.13% menthol, 12.82% sodium chloride, 10.26% anhydrous citric acid, 5.13% glycerol, 2.56% titanium dioxide, and 51.28% hydroxypropyl methylcellulose, wherein the percentage refers to the percentage of the mass of each of the components relative to the total mass of the risperidone solid oral film;

formula 7: 4.88% risperidone, 7.32% sucralose, 4.88% menthol, 12.19% sodium chloride, 14.63% anhydrous citric acid, 4.88% glycerol, 2.44% titanium dioxide, and 48.78% hydroxypropyl methylcellulose, wherein the percentage refers to the percentage of the mass of each of the components relative to the total mass of the risperidone solid oral film;

formula 8: 4.44% risperidone, 6.67% sucralose, 4.44% menthol, 11.12% sodium chloride, 22.22% anhydrous citric acid, 4.44% glycerol, 2.23% titanium dioxide, and 44.44% hydroxypropyl methylcellulose, wherein the percentage refers to the percentage of the mass of each of the components relative to the total mass of the risperidone solid oral film;

formula 9: 4.00% risperidone, 6.00% sucralose, 4.00% menthol, 10.00% sodium chloride, 30.00% anhydrous citric acid, 4.00% glycerol, 2.00% titanium dioxide, and 40.00% hydroxypropyl methylcellulose, wherein the percentage refers to the percentage of the mass of each of the components relative to the total mass of the risperidone solid oral film;

formulas 10-12: 2.01% risperidone, 3.01% sucralose, 40.16% microcrystalline cellulose, 5.02% sodium chloride, 6.63% citric acid monohydrate, 10.04% glycerol, 3.01% titanium dioxide, and 30.12% hydroxypropyl methylcellulose, wherein the percentage refers to the percentage of the mass of each of the components relative to the total mass of the risperidone solid oral film;

formula 13: 0.56% risperidone, 6.76% sucralose, 4.51% menthol, 11.27% sodium chloride, 9.86% citric acid monohydrate, 4.51% glycerol, 15.21% microcrystalline cellulose, 2.25% titanium dioxide, and 45.07% hydroxypropyl methylcellulose, wherein the percentage refers to the percentage of the mass of each of the components relative to the total mass of the risperidone solid oral film;

formula 14: 4.51% risperidone, 6.76% sucralose, 4.51% menthol, 11.27% sodium chloride, 9.86% citric acid monohydrate, 4.51% glycerol, 11.26% microcrystalline cellulose, 2.25% titanium dioxide, and 45.07% hydroxypropyl methylcellulose, wherein the percentage refers to the percentage of the mass of each of the components relative to the total mass of the risperidone solid oral film;

formula 15: 0.54% risperidone, 6.44% sucralose, 4.29% menthol, 10.73% sodium chloride, 14.16% citric acid monohydrate, 4.29% glycerol, 14.48% microcrystalline cellulose, 2.15% titanium dioxide, and 42.92% hydroxypropyl methylcellulose, wherein the percentage refers to the percentage of the mass of each of the components relative to the total mass of the risperidone solid oral film;

formula 16: 4.29% risperidone, 6.44% sucralose, 4.29% menthol, 10.73% sodium chloride, 14.16% citric acid monohydrate, 4.29% glycerol, 10.73% microcrystalline cellulose, 2.15% titanium dioxide, and 42.92% hydroxypropyl methylcellulose, wherein the percentage refers to the percentage of the mass of each of the components relative to the total mass of the risperidone solid oral film.

6. The risperidone solid oral film as claimed in claim 1, wherein the risperidone solid oral film has a dry film thickness of 10 μm-200 μm.

7. A preparation method for the risperidon solid oral film as claimed in any one of claims 1-6, comprising the following steps:

step 1: mixing two or more of a solvent, a flavoring agent, a stabilizer, a plasticizer, a colorant, a filler, and a film-forming material according to a formula amount, performing electrically stirring or vacuum-emulsifying until uniform, and performing vacuum deaeration to obtain a drug-containing gel solution;
step 2: adding the drug-containing gel solution obtained in step 1 into a film preparation machine for film coating and drying to obtain a dry film intermediate;
step 3: cutting and packaging the dry film intermediate obtained in step 2 to obtain the risperidone solid oral film.

8. Use of the risperidone solid oral film as claimed in any one of claims 1-6 for manufacturing a medicament for treating and/or preventing a neuropsychiatric disease and/or a psychiatric disorder, wherein

preferably, the neuropsychiatric disease and the psychiatric disorder are selected from the group consisting of schizophrenia, psychotic disorder, Alzheimer's disease, frontotemporal dementia, vascular dementia, dementia with Lewy bodies, senile dementia, mild cognitive impairment, benign amnesia, closed head trauma, autism spectrum disorder, Asperger's disease, fragile X syndrome, attention deficit hyperactivity disorder, attention deficit disorder, obsessive compulsive disorder, tic disorder, learning disorder in children, premenstrual syndrome, depression, major depression, anhedonia, suicidal ideation and/or behavior, bipolar disorder, anxiety disorder, panic disorder, post-traumatic stress disorder, chronic mild and unpredictable stress, eating disorder, addiction disorder, personality disorder, Parkinson's disease, Huntington's disorder, multiple sclerosis, amyotrophic lateral sclerosis, ataxia, Friedreich's ataxia, Tourette's syndrome, nocturnal enuresis, non-epileptic seizure, blepharospasm, Duchenne muscular dystrophy, stroke, chronic pain, neuropathic pain, hyperalgesia, allodynia, diabetic polyneuropathy, epileptic seizure, and epilepsy.

9. Use of the risperidone solid oral film as claimed in any one of claims 1-6 for manufacturing a pharmaceutical formulation, wherein

preferably, the pharmaceutical formulation is a pharmaceutical formulation for treating and/or preventing a neuropsychiatric disease and/or a psychiatric disorder; preferably, the neuropsychiatric disease and the psychiatric disorder are selected from the group consisting of schizophrenia, psychotic disorder, Alzheimer's disease, frontotemporal dementia, vascular dementia, dementia with Lewy bodies, senile dementia, mild cognitive impairment, benign amnesia, closed head trauma, autism spectrum disorder, Asperger's disease, fragile X syndrome, attention deficit hyperactivity disorder, attention deficit disorder, obsessive compulsive disorder, tic disorder, learning disorder in children, premenstrual syndrome, depression, major depression, anhedonia, suicidal ideation and/or behavior, bipolar disorder, anxiety disorder, panic disorder, post-traumatic stress disorder, chronic mild and unpredictable stress, eating disorder, addiction disorder, personality disorder, Parkinson's disease, Huntington's disorder, multiple sclerosis, amyotrophic lateral sclerosis, ataxia, Friedreich's ataxia, Tourette's syndrome, nocturnal enuresis, non-epileptic seizure, blepharospasm, Duchenne muscular dystrophy, stroke, chronic pain, neuropathic pain, hyperalgesia, allodynia, diabetic polyneuropathy, epileptic seizure, and epilepsy.

10. The use as claimed in claim 9, wherein the pharmaceutical formulation is a risperidone fast-soluble and/or immediate-release solid oral film administered via the gastrointestinal tract.

RRT1.14 impurity under different citric acid dosages

FIG. 1

## INTERNATIONAL SEARCH REPORT

| | |
|---|---|
| International application No. | **PCT/CN2024/118533** |

**A. CLASSIFICATION OF SUBJECT MATTER**

A61K9/70(2006.01)i; A61K31/519(2006.01)i; A61P25/18(2006.01)i

According to International Patent Classification (IPC) or to both national classification and IPC

**B. FIELDS SEARCHED**

Minimum documentation searched (classification system followed by classification symbols)

IPC: A61K, A61P

Documentation searched other than minimum documentation to the extent that such documents are included in the fields searched

Electronic data base consulted during the international search (name of data base and, where practicable, search terms used)

CNABS, CNTXT, CNKI, DWPI, VEN: 上海云晟研新生物科技有限公司, 上海博志研新药物研究有限公司, 博志研新泰州药物技术有限公司, 应述欢, 付俊, 金海刚, 郭桢, 王婷婷, 利培酮, 膜剂, 口溶, 口崩, 口腔, 分散膜, 矫味剂, 稳定剂, 增塑剂, 填充剂, 着色剂, 成膜材料, 成膜剂, risperidone, film, oral dissolving film, oral instant film, orodispersible film, oral, mouth, flavoring agent, stabilizer, plasticizer, filler, coloring agent, film forming agent

**C. DOCUMENTS CONSIDERED TO BE RELEVANT**

| Category* | Citation of document, with indication, where appropriate, of the relevant passages | Relevant to claim No. |
|---|---|---|
| X | 张桦 等 (ZHANG, Hua et al.). "利培酮口溶膜的制备及体内外评价 (Preparation and In Vitro/In Vivo Evaluation of Risperidone Orodispersible Films)" 中国医药工业杂志 (Chinese Journal of Pharmaceuticals), Vol. 48, No. 3, 31 December 2017 (2017-12-31), pages 406-412 page 406, right-hand column, paragraph 1, page 408, columns 2.4 and 2.5.1, page 409, column 2.6, and page 411, column 3.1 | 1-10 |
| X | CN 107028917 A (WUHAN HUAXIA INSTITUTE OF TECHNOLOGY) 11 August 2017 (2017-08-11) description, paragraphs [0002] and [0012]-[0035], and embodiments 1-6 | 1-10 |
| X | CN 108685876 A (QILU PHARMACEUTICAL CO., LTD.) 23 October 2018 (2018-10-23) description, paragraph [0006], and embodiment 1 | 1-10 |
| X | CN 101632651 A (HEBEI UNIVERSITY OF SCIENCE AND TECHNOLOGY) 27 January 2010 (2010-01-27) claims 1-10, and description, page 1, lines 6-13, and embodiments 5-6 | 1-10 |

| ☑ Further documents are listed in the continuation of Box C. | ☑ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **16 December 2024** | **03 January 2025** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
|---|
| **PCT/CN2024/118533** |

**C.      DOCUMENTS CONSIDERED TO BE RELEVANT**

| Category* | Citation of document, with indication, where appropriate, of the relevant passages | Relevant to claim No. |
|---|---|---|
| X | CHEN, Fang et al. "Evaluation of the Impacts of Formulation Parameters on the Pharmacokinetics and Bioequivalence of Risperidone Orodispersible Film: a Physiologically Based Pharmacokinetic Modeling Approach"<br>*AAPS PharmSciTech*, Vol. 21, 27 August 2020 (2020-08-27), pages 1-12<br>    page 1, right-hand column, lines 2-15, and page 2, right-hand column, lines 5-22 | 1-10 |
| A | US 2019336453 A1 (BONAYU LIFESCIENCES PRIVATE LTD.) 07 November 2019 (2019-11-07)<br>    embodiments 1-6 | 1-10 |
| A | 吴小玉 等 (WU, Xiaoyu et al.). "利培酮口溶膜剂的制备及质量评价 (Preparation and Quality Evaluation of Risperidone Oral Soluble Films)"<br>*中国医院药学杂志 (Chinese Journal of Hospital Pharmacy)*,<br>Vol. 37, No. 22, 30 November 2017 (2017-11-30), pages 2251-2254<br>    page 2252, columns 2.1-2.3 | 1-10 |
| A | SAHU, H. et al. "Formulation and Evaluation of Risperidone Loaded Mouth-Dissolving Film"<br>*Research Journal of Pharmacy and Technology*,<br>Vol. 11, No. 7, 31 July 2018 (2018-07-31), pages 2922-2925<br>    page 2923, table 2 | 1-10 |

Form PCT/ISA/210 (second sheet) (July 2022)

## INTERNATIONAL SEARCH REPORT
### Information on patent family members

International application No.

**PCT/CN2024/118533**

| Patent document cited in search report | | | Publication date (day/month/year) | Patent family member(s) | | | Publication date (day/month/year) |
|---|---|---|---|---|---|---|---|
| CN | 107028917 | A | 11 August 2017 | None | | | |
| CN | 108685876 | A | 23 October 2018 | None | | | |
| CN | 101632651 | A | 27 January 2010 | None | | | |
| US | 2019336453 | A1 | 07 November 2019 | WO | 2018127938 | A1 | 12 July 2018 |
| | | | | EP | 3565532 | A1 | 13 November 2019 |
| | | | | EP | 3565532 | A4 | 28 October 2020 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202311186977
- CN 101632651 A
- CN 103349657 A
- CN 108685876 A
- CN 104546806 A
- CN 113842376 A
- CN 107028917 B
- CN 107028917 A

**Non-patent literature cited in the description**

- Chinese Pharmacopoeia. 2020, vol. IV
- **LI PAN** ; **HAN XUE** ; **LIN JUNZHI** ; **JIANG HONG** ; **YANG MING** ; **ZHANG DINGKUN** ; **HAN LI**. Application and Development of Volunteer Sensory Test in Drug Taste Evaluation [J].. *Chinese Pharmaceutical Journal*, 2017, vol. 52 (22), 1971-1975